# EUROPEAN PATENT APPLICATION

(11) **EP 2 133 358 A1**
(43) Date of publication of application: **16.12.2009**
(21) Application number: 08010548.9
(22) Date of filing: 10.06.2008
(51) Int. Cl.: C07K 14/175, A61K 39/12

(54) **Rift valley fever virus-like particles and their use for immunization and as test system**

(71) Applicant: Universitätsklinikum Freiburg, 79106 Freiburg (DE)
(72) Inventor: Weber, Friedemann, 79114 Freiburg (DE); Habjan, Matthias, 79104 Freiburg (DE); Spiegel, Martin, 37075 Göttingen (DE); Penski, Nicola, 79104 Freiburg (DE)
(74) Representative: Keller, Günter

(57) **Abstract**

Disclosed is a method for producing virus-like particles (VLP) comprising
a) transfecting a mammalian cell line with several independent vectors comprising
aa) a vector containing at least a substantial part of the M gene from Rift Valley Fever Virus,
bb) a vector containing at least a substantial part of the L gene from Rift Valley Fever Virus,
cc) a vector containing at least a substantial part of the N gene from Rift Valley Fever Virus and
dd) a vector containing a multicloning site flanked by the non-coding 5'-and 3'-ends of the M-segment of Rift Valley Fever Virus, and

b) culturing the transfected mammalian cell line under suitable conditions and obtaining the VLPs from the supernatant of the transfected cultured cell lines.

Said VLPs can be used for vaccination and in methods for testing the antiviral activity of compounds.

## Description

Rift Valley fever Virus (RVFV) is a serious emerging pathogen affecting humans and livestock in sub-Saharan Africa, Egypt, Yemen, and Saudi Arabia. Since the first description of an outbreak in Kenya in 1931, there have been recurrent epidemics, killing thousands of animals, hundreds of humans, and causing significant economic losses. To animals, RVFV is mainly transmitted by mosquitoes, but humans are often infected by close contact with infected animals' material. A wide array of symptoms is connected with the human disease, ranging from an uncomplicated acute febrile illness to retinitis, hepatitis, meningoencephalitis, severe hemorrhagic disease, and death. The severity of RVFV zoonosis as well as the capability to cause major epidemics in livestock and humans have prompted authorities to list RVFV as a notifiable disease and a potential biological weapon.

RVFV belongs to the genus Phlebovirus, family *Bunyaviridae*. Bunyaviruses are enveloped and have a tri-segmented single-stranded RNA genome of negative or ambisense polarity, replicate in the cytoplasm, and bud into the Golgi apparatus. They encode five common structural proteins:
the viral polymerase (L) on the large (L) segment,
two glycoproteins (Gn and Gc) and the 78kD protein on the medium (M) segment, and
the viral nucleocapsid protein (N) on the smallest (S) segment.

RVFV additionally expresses two nonstructural proteins, encoded on the M segment (termed NSm) and the S segment (termed NSs). These accessory proteins are dispensable for viral multiplication in cell culture, but play important roles for pathogenesis *in vivo*. In particular, the NSm and 78kD proteins were found to enhance intrahost viral spread, whereas NSs is important to suppress the antiviral interferon system.

The general features of RVFV transcription and replication are similar to those of other negative-stranded RNA viruses. The viral genomic RNA (vRNA) segments contain untranslated regions (UTRs) on both the 3' and the 5' ends that serve as promoters for replication of the segment and transcription of the encoded reading frames. The vRNAs are encapsidated by N protein and associate with L protein both intracellularly and in the virion, and only these ribonucleoprotein particles (RNPs) are functional templates for mRNA synthesis and RNA replication by the viral polymerase.

After Gn/Gc-mediated entry into the cytoplasm, the negative-sense vRNAs of RVFV are transcribed into mRNAs by the RNP-associated L and N proteins ("primary transcription"). The products of these immediate early mRNAs then provide the machinery for replication of the genome and further mRNA synthesis ("secondary transcription"), and later drive the packaging of newly assembled RNPs into budding particles. A specific feature of bunyaviruses is that their mRNA synthesis is strictly dependent on host translation. Most likely, ongoing translation prevents premature termination of transcription by secondary structures building up on the nascent mRNA.

The infection cycle of bunyaviruses is strongly inhibited by the interferon-induced MxA protein of humans. The cytoplasmatically located MxA is capable of binding recombinant N protein of RVFV and several other bunyaviruses, and to sequester it to perinuclear complexes. It remained unclear, however, whether MxA solely cuts bunyaviruses off the supply of N protein, or whether it also affects assembled RNPs and inhibits their transcriptional activity.

Recently, reverse genetic systems to rescue infectious RVFV particles entirely from cloned cDNA plasmids have been developed (Ikegami, Journal of Virology [May 2005], pp 5606-5615; Gerrard et al., Virology 359, 459-65 [2007]). These systems allow the targeted manipulation of the viral genome and proved to be extremely useful to demonstrate the contribution of NSs and the NSm proteins to viral pathogenesis and to identify transcriptional termination signals.

However, research on RVFV is hampered and restricted since the pathogen can only be handled under biosafety level 3 (BSL3) conditions. Therefore, it would be desirable to have tools at hand which allow to study aspects of RVFV infection cycle and the host response under non-BSL3 conditions.

The establishment of a RVFV reverse genetics system which recapitulates the first steps of the replication cycle in a modular manner is disclosed. A minireplicon system was developed to measure RVFV polymerase activity. Additional expression of viral glycoproteins allowed efficient and helper-free packaging of minireplicon RNPs into virus-like particles (VLPs) which were released into the medium. By incubating naive cells with VLPs, the first steps of RVFV infection are reconstituted. Importantly, the system allowed to demonstrate that the L polymerase complexed with incoming RNPs is highly active in primary transcription and, if both L and the nucleoprotein N are additionally provided *in trans*, replication as well. The suitability of our modular approach was further validated by testing whether the antiviral protein MxA inhibits the primary and/or the secondary transcription of RVFV. The cytoplasmic human MxA which is encoded by chromosome 21 has antiviral activity against several viruses, in particular against Bunyaviruses.

The present invention discloses a method for producing virus-like particles whereby a mammalian cell line, preferably a human cell line, is transfected with several independent vectors. The cell lines are preferably derived from human origin since the virus-like particles are preferably used as vaccine which provides immunity against an infection of Rift Valley Fever Virus. The host cell is higher eukaryotic (for example, all mammals, including but not limited to mouse and human) and may be used for expression of the desired coding sequences when appropriate control sequences which are compatible with the designated host are used.

Mammalian cell lines available as hosts for expression of cloned genes are known in the art and include many immortalized cell lines available from the American Type Culture Collection (ATCC), such as HEp-2, CHO cells, Vero cells, baby hamster kidney (BHK) cells and COS cells or BSR-T7/5 cells, to name a few. Suitable promoters are also known in the art and include viral promoters such as that from SV40, Rous sarcoma virus (RSV), adenovirus (ADV), bovine papilloma virus (BPV), and cytomegalovirus (CMV).

Mammalian cells may also require terminator sequences, poly A addition sequences, enhancer sequences which increase expression, or sequences which cause amplification of the gene. These sequences are known in the art.

Preferred cell lines are selected from the group consisting of cell lines having the well-known scientific designations HEK 293, 293T, Vero, HeLa, COS-1, A549, Huh-7, Huh-7.5, Hep2G, Jurkat and/or BSR-T 7/5.

The advantage of the present method is that the cell lines do not contain infections with Rift Valley Fever Virus or inactivated RVFV virus at any time during the production of VLPs. Therefore, there is no risk that by recombinational events viable and infectious dangerous viruses emerge. The genes M, L and N, respectively, are cloned into a vector which can transiently express the open reading frames of the M, L and N genes. It is not necessary that the complete M, L and/or N gene from RVFV is contained within the vectors. It is sufficient if the sequence coding for M, L and N are present in a form which shows a homology of at least 80%, preferably at least 90% to the gene calculated over the whole length of the gene. According to the present invention at least a substantial part of the gene coding for M, L and/or N is present in the vector.

Furthermore the cell line is transfected with a vector which is designated in the present application as a minireplicon plasmid. The minireplicon plasmid contains a multicloning site flanked by the non-coding 5'- and 3'- ends of the M segment of Rift Valley Fever Virus. The minireplicon plasmid is not self-replicating but requires the coexpression of N and L genes which lead to transcription and replication of the virus like RNA encoded by the minireplicon plasmid.

The advantage of this minireplicon is that an artificial gene segment can be inserted into the virus-like particles. Moreover, by packaging a suitable foreign gene into the VLP with the help of the minireplicon system it is possible to enhance the immunological activity and to improve the efficacy of VLPs in the immunization of patients and/or animals.

The inserted gene may be a gene obtained from RVFV like for example a gene coding for the Gn and/or Gc protein or the nucleoprotein N or the polymerase L. Alternatively the gene may code for a protein which stimulates the immune response. It may for example code for a ligand to which a receptor of immune cells may bind or alternatively interferons like IFN-beta, immuncytokines like IL-2, CXCL-10. In the experiments of the present application the minireplicon contained a luciferase gene obtained from *Renilla reniformis* as model system in order to show that the gene contained within the minireplicon is expressed. In the vaccination experiments VLPs comprising an artificial gene segment preferably encoding the RVFV N gene fragment were used.

In a preferred embodiment the minireplicon is transcribed with the help of an RNA-polymerase I promoter which may be obtained from human origin and/or mouse. Alternatively the use of a T7 polymerase promoter is also possible.

The invention relates in another embodiment to virus-like particles (VLP) obtainable by the method of the present invention. Preferably such VLPs are used in pharmaceutical compositions as vaccine against an infection of RVFV. The VLPs of the present invention have several advantages. Since the VLPs are produced in mammalian, preferably human cell lines they have a glycosylation pattern on the protein which is specific for human. Therefore, there is no risk that undesired immunological reactions may occur. Such risk can, however, not be excluded when products are used which are produced in other organisms like plant cells and/or insect cells. The folding of the glycoproteins of the VLPs according to the present invention corresponds exactly to the usual folding of human proteins. Therefore, it can be concluded that immunological reactions of the immunized individual are specifically directed against proteins contained within the VLPs obtained from Rift Valley Fever Virus like N, L, G proteins.

In an alternative embodiment the virus-like particles are used for the vaccination of animals, in particular sheep and/or cattle. Since the virus can cause substantial losses in agriculture there is a demand to provide an efficient system for vaccination of animals like cows, sheep, horses, goats or camels. For preparing suitable VLPs it is preferred to use cell lines of the same species to be vaccinated. For the vaccination of cows, preferably bovine cell lines are used.

An important advantage of the VLPs of the present application is that their proteins correspond exactly to the proteins derived from the virus without, however, the risk that an attenuated virus used for immunization may be reactivated by undesired recombination events resulting in undesired side-effects.

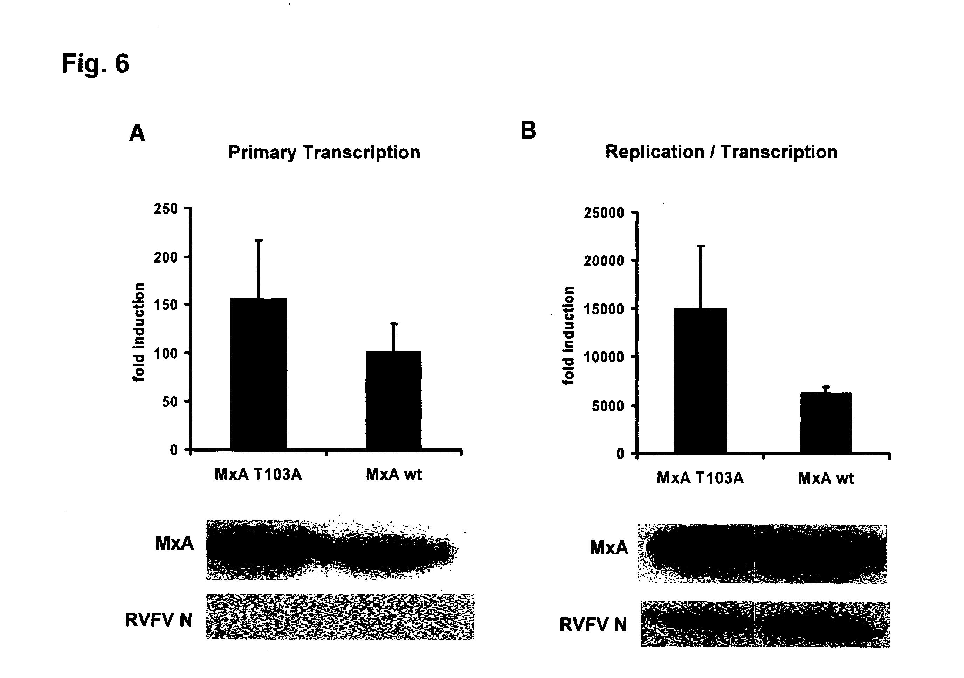

The virus-like particles of the present invention can be used in a method for testing whether an agent has antiviral activity against Rift Valley Fever Virus. This test method is a kind of screening method wherein VLPs prepared according to the teaching of the present application are brought into contact with a mammalian cell line which can be infected by the VLPs. Additionally, an agent which is supposed to have antiviral activity is added to the test sample. In a comparative test the same amount of the VLPs and cells of the same cell line are brought together whereby the agent to be tested is replaced by a suitable control. The control can be either the corresponding amount of buffer without any agent or it can be a corresponding compound which does not have an antiviral activity. One example of the agent to be tested is the antiviral human protein MxA. In the examples (see Figure 6) the human protein MxA has been tested in an active form compared with an inactive form.

Furthermore, the testing method is suitable for determining the activity of neutralizing antibodies having activity against RVFV or for testing any chemically synthesized compound or compounds derived from naturally occurring substances which are supposed to have antiviral activity. The advantage of the test method is that it can be performed under relatively low security conditions, since no viable virus is used. Furthermore, the test can be easily automated, standardized and a high throughput of compounds to be screened is possible.

A screening test for agents having antiviral activity can be performed in the following manner. A suitable cell line (e.g. BHK cells) were seeded in equal amounts into plates having 96 wells. After growth of the cells for one day, about 50 µl of a VLP solution containing about 10⁴ active VLPs are dispensed in each well and the cells are incubated for one hour. Thereafter to each well an aliquot of about 50 µl medium is added whereby the test solution contains the agent to be tested and the control does not contain the agent to be tested. After incubation over night the cells are suspended in 20 µl passive lysis buffer and the *Renilla* activity is measured. By using microtiter plates having for example 96 wells it is easy to perform the test in an automated manner by using suitable laboratory automated devices.

The present invention is shown in the enclosed Figures and preferred embodiments are described in the examples.

### Description of Figures

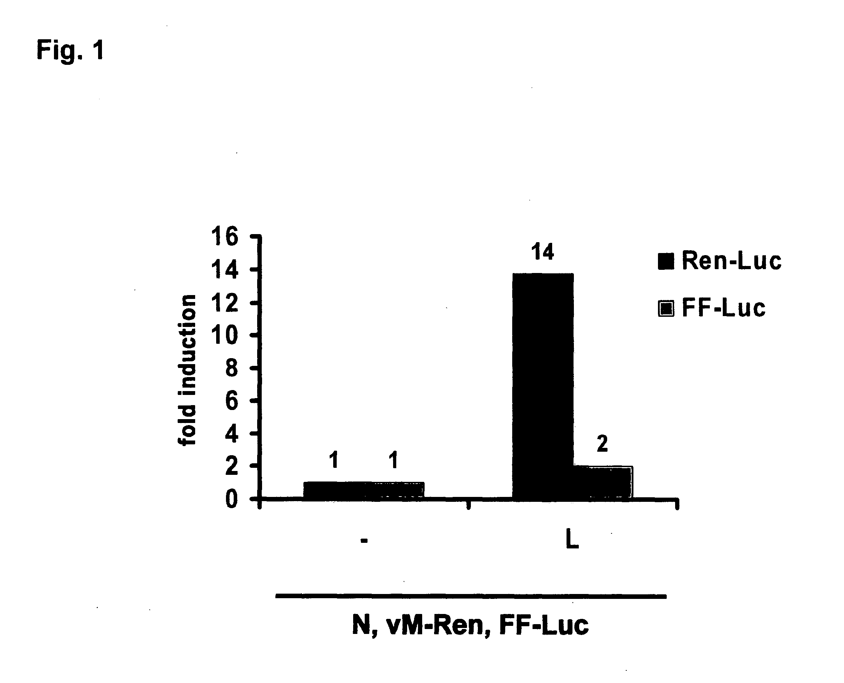

**Fig. 1****: Minireplicon system for RVFV.**
   Human 293T cells were transfected with expression plasmids for the nucleoprotein N and the viral polymerase L, together with a minireplicon construct containing the Ren-Luc reporter gene in antisense flanked by the untranslated regions of the M segment (vM-Ren). As a negative control the L expression plasmid was omitted. An FF-Luc plasmid was cotransfected to control for background expression. Cell lysates were assayed 24 h post transfection for Ren-Luc and FF-Luc activity. Luciferase counts were normalized to the experiment without L, numbers on top of each column indicating fold induction. Data from a representative experiment are shown.

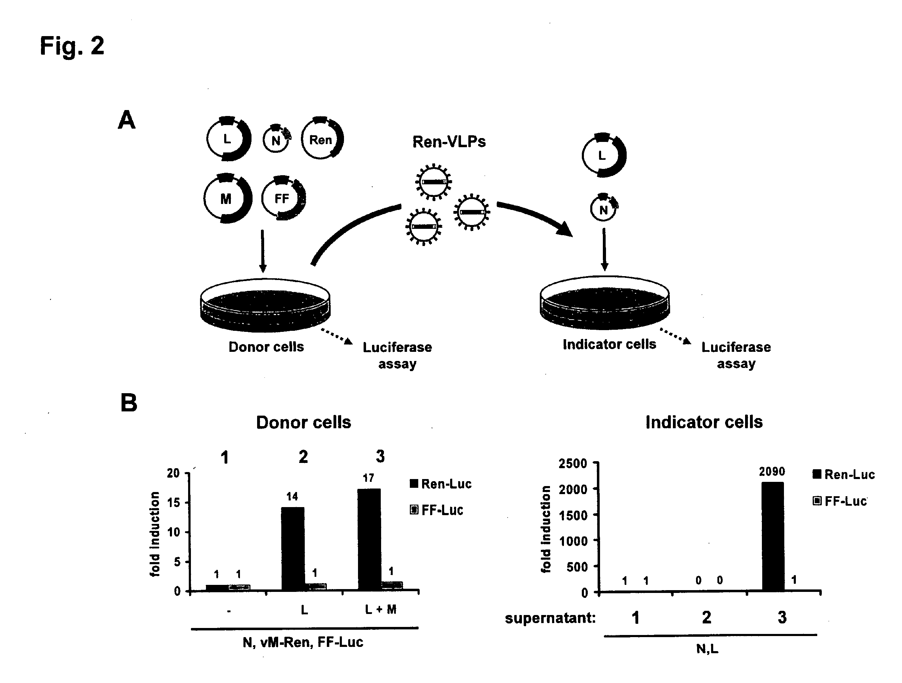

**Fig. 2****: Formation of RVFV VLPs.**
   (A) Outline of the procedure used for generation of VLPs. Donor cells were transfected with expression plasmids for N, L and the viral glycoproteins (M), together with the reporter minireplicon construct vM-Ren. Release of VLPs into the supernatant of donor cells was detected by transfer of supernatants on indicator cells expressing both N and L.
   (B) Luciferase activities in donor and indicator cells. 293T cells (donor cells) were transfected with minireplicon system plasmids alone as described in Fig. 1 (left panel, columns 1 and 2), or together with an M segment expression plasmid (left panel, column 3). Supernatants were harvested 24 h later and used to infect BSR-T7/5 indicator cells (right panel). FF-Luc and Ren-Luc activities in donor cells were measured upon removal of supernatants, and indicator cells were analyzed 24 h post infection. Luciferase counts were normalized to the experiment where L has been omitted, and numbers on top of each column display fold induction. Data from a representative experiment are shown.

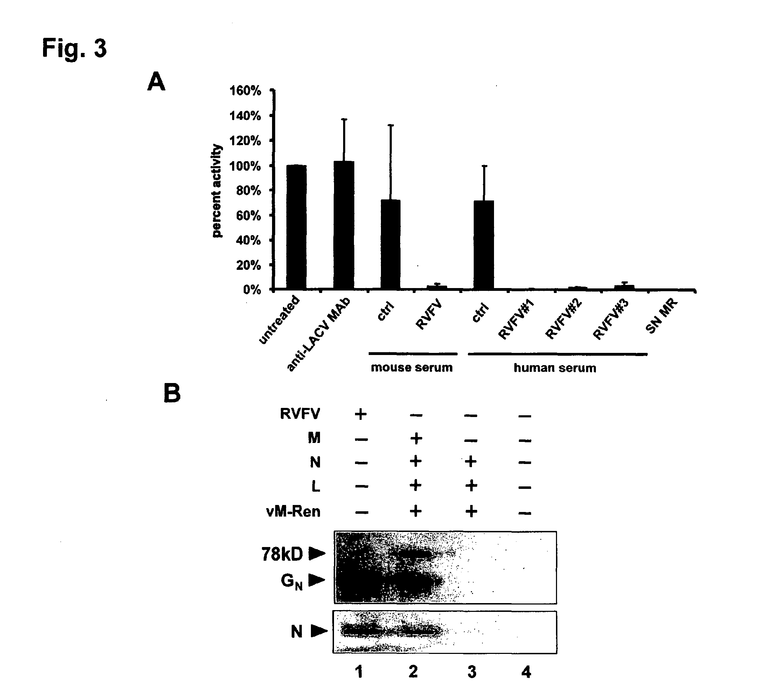

**Fig. 3****: VLPs resemble authentic RVFV particles.**
   (A) Neutralisation of VLPs by RVFV-specific antisera. VLP-containing supernatants were incubated with 5 µl of mouse or human antisera specific for RVFV for 1 hour at 37°C. As controls, 5 µl of a non-seroconverted mouse and human (ctrl) or of a monoclonal antibody against La Crosse bunyavirus glycoprotein Gc were used. Cells were then infected with antibody-treated or untreated VLP preparations, or the supernatant from minireplicon-expressing cells (SN MR). Ren-Luc activity was measured 24 hours later and is shown as percent activity of untreated VLPs. Error bars show standard deviations from three independent experiments. (B). Western blot analysis of RVF virus particles and VLPs. Concentrated supernatants from RVFV-infected cells (lane 1) and 293T cells transfected with different plasmid combinations (lane 2 to 4) were analyzed by Western blotting using a monoclonal antibody specific for RVFV Gn and 78kD protein (upper panel), and an antibody against the nucleoprotein N (lower panel).

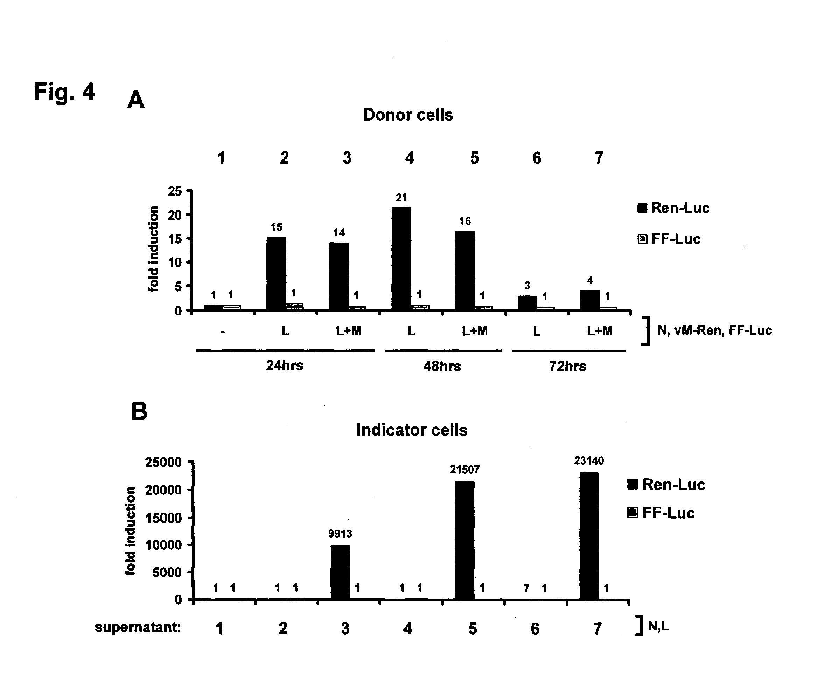

**Fig. 4****: Optimisation of VLP production.**
   VLPs were generated as described in Fig. 2, and VLP release from donor cells was followed over the course of three days. Supernatants from parallel dishes were harvested at the timepoints indicated and used to infect indicator cells expressing N and L. (A) Ren-Luc and FF-Luc activities in donor cells were measured upon removal of supernatant. (B) Analysis of luciferase activities in indicator cells was performed 24 h post-infection. Luciferase counts were normalised to the experiment where L has been omitted. Data from a representative experiment are shown, numbers on top of each column displaying fold induction.

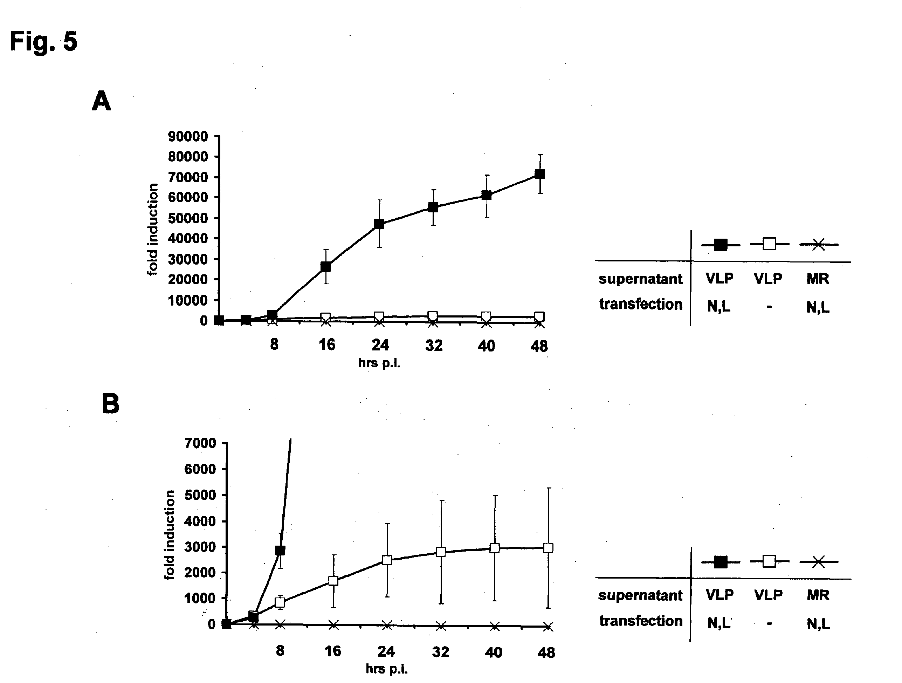

**Fig. 5****: Primary and secondary transcription in VLP-infected cells.**
   (A). BSR-T7/5 cells were either mock transfected (open squares), or transfected with N and L expression plasmids (filled squares) prior to infection with VLPs harbouring Ren-Luc RNPs. As a negative control, cells transfected with N and L plasmids were infected with supernatants from minireplicon-expressing donor cells (crosses). Ren-Luc activities in VLP-infected cells were then measured at different timepoints over the course of 48 h. (B). The same data is shown as in A, but with the y-axis adjusted to visualise activity of VLPs in naive cells. Error bars show standard deviations from three independent experiments.
**Fig. 6****: Inhibition of both RVFV primary and secondary transcription by human MxA.**
   The influence of human MxA on RVFV primary transcription was analyzed by transfecting BSR-T7/5 cells with 1.5µg of an expression construct for wt MxA (A). To study the effect of MxA on RVFV replication and secondary transcription, the same amount of plasmid for wt MxA was cotransfected together with 0.5µg of each N and L (B). The inactive MxA-mutant T103A was used as a negative control. Twenty-four hours post transfection, cells were infected with VLPs harbouring Ren-Luc RNPs. *Renilla* luciferase activities in cell lysates were measured 24 hours later. Equal amounts of these lysates were used for Western Blot analysis to confirm expression of both MxA and RVFV N. Data are shown from three independent experiments performed in parallel, error bars indicating standard deviations.

### Example 1: Construction of Plasmids

All plasmids were generated using standard molecular cloning techniques and confirmed by DNA sequencing. PCR was carried out with AccuPrime Pfx DNA Polymerase (Invitrogen; for cloning purposes) or Taq DNA Polymerase (Eppendorf; for diagnostic purposes and addition of single adenosines for TA cloning). TA cloning was done using the pcDNA3.1/V5-His TOPO TA Expression Kit (Invitrogen) according to the manufacturers instructions. The preparation of RVFV first-strand cDNA was performed as described previously for La Crosse virus (Blakqori et al., J.Gen.Virol. 84 (2003), pp 1207-1214).

Viral genes were amplified from the first-strand cDNA using specific primers as shown in Table 1.

**Table 1a: DNA oligonucleotide primers**

| **Name** | **SEQ ID NO.** | **Sequence (5' to 3')^{a}** | **Segment/Gene** | **Nucleotides^{b}.^{c}** |
|---|---|---|---|---|
| RVFV_SapI_L1for | 1 | GACAGAGCTCTTC**ATCATGGATTCTATATTATCAAAACAGCTG** | L gene | 16-45 |
| RVFV_L1rev | 2 | **TGAGGCATCCATTGCTGC** | L gene | 1959-1942 |
| RVFV_L2for | 3 | **GCTGGGCCTTTGATCTCTC** | L gene | 1727-1745 |
| RVFV_L2rev | 4 | **CTCCCGATGACCATCCAG** | L gene | 3159-3142 |
| RVFV_L3for | 5 | **GAGGAAAGAATTGTTCAATCGG** | L gene | 2818-2839 |
| RVFV_L3rev | 6 | **AATGGTCACGGATAACCATAGC** | L gene | 4867-4846 |
| RVFV_L4for | 7 | **AAGCAACTCTAGCTCACACCCC** | L gene | 4712-4733 |
| RVFV_L4rev_SapI | 8 | GACAGAGCTCTTC**TGGTCTTAGCCTAGCATGTCATC** | L gene | 6302-6280 |
| RVFV_SapI_M1for | 9 | GACAGAGCTCTTC**TAAATGTATGTTTTATTAACAATTCTAATCTCG** | M gene | 18-50 |
| RVFV_M1rev | 10 | **TCAAACAAGCCTCTGCCC** | M gene | 2217-2200 |
| RVFV_M2for | 11 | **GTGAACAGGGAAATAGGATGG** | M gene | 1956-1976 |
| RVFV_M2rev_SapI | 12 | GACAGAGCTCTTC**CTGATCTATGAGGCCTTCTTAGTG** | M gene | 3619-3596 |

| | | | | |
|---|---|---|---|---|
| ^{a}nucleotides corresponding to RVFV sequences are in boldface letters ^{b}nucleotide numbers according to the database entries DQ375403 (L segment), DQ380206 (M segment), and DQ380151 (S segment) ^{c}hybridization sites for reverse primers are indicated by reverse order numbering | | | | |

**Table 1b: DNA oligonucleotide primers**

| **Name** | **SEQ ID NO.** | **Sequence (5' to 3')^{a}** | **Segment/Gene** | **Nucleotides^{b,c} -** |
|---|---|---|---|---|
| RVFV_SapI_Sfor | 13 | GACAGAGCTCTTC**ATAATGGACAACTATCAAGAGCTTGC** | N gene | 36-61 |
| RVFV-C13_Srev_SapI | 14 | GACAGAGCTCTTC**CAAGCAGCAAAAGAGGACATTTC** | N gene | 1062-1040 |
| RVFV_cMPro1for | 15 | GACAGACGTCTCCTATAG**ACACAAAGACGGTGCATTAAAT**GAAGAGCGGTACCGCTCTTC**ATCAGTACGTGTAAAAGCAA** | M UTR | 1-//-3634 |
| RVFV_cMPro2rev | 16 | **TGACCTATCTGTACAATACTTACATAATTAGGTTTGCTTATGTCTACTTATTTCAACATATTGCTTTTACACGTACTGAT** | M UTR | 3694-3615 |
| RVFV_cMPro3for | 17 | **AGTATTGTACAGATAGGTCAAATTATTGGAATATCCAAGCTTAGAAACTTATGCAATAATACTTTAGATGTAAGCTTAGT** | M UTR | 3675-3754 |
| RVFV_cMPro4rev | 18 | **ACTAGAATATTCACAAGCACTTGAGACTGCTGCTGCCTCACCCCACCACCCCAAATTACAACTAAGCTTACATCTAAAGT** | M UTR | 3814-3735 |
| RVFV_cMPro5for | 19 | **GTGCTTGTGAATATTCTAGTTGGCGTAATCGTCTTTTGCCAGATTAGCTGGGAATTAAACTAACTCTTTGAAGTTGCACC** | M UTR | 3795-3874 |
| RVFV_cMPro6rev | 20 | TCTGTCCGTCTCCACCC**ACACAAAGACCGGTGCAACTTCAAAGAGTTA** | M UTR | 3885-3855 |
| RVFV_vMPro_pHH21for | 21 | GACAGACGTCTCCTATT**ACACAAAGACCGGTGCAACTTC** | - | - |
| RVFV_vMPro_pHH21rev | 22 | GACAGACGTCTCCGGG**ACACAAAGACGGTGCATTAA** | - | - |
| RVFV_MRen_SapIfor | 23 | GACAGAGCTCTTCAAA*ATGACTTCGAAAGTTTATGATCCAG* | - | - |
| RVFV_MRen__SapIrev | 24 | GACAGAGCTCTTCTTGAC*TTATTGTTCATTTTTGAGAACTCGC* | - | - |

| | | | | |
|---|---|---|---|---|
| ^{a}nucleotides corresponding to RVFV sequences are in boldface letters ^{b}nucleotide numbers according to the database entries DQ375403 (L segment), DQ380206 (M segment), and DQ380151 (S segment) ^{c}hybridization sites for reverse primers are indicated by reverse order numbering | | | | |

The constructs pI.18_RVFV_L (SEQ ID NO:25), pI.18_RVFV_M (SEQ ID NO:26) and pI.18-RVFV_N (SEQ ID NO:27) contained the appropriate coding sequences subcloned into the eukaryotic high-level expression plasmid pI.18 (kindly provided by Jim Robertson, National Institute for Biological Standards and Control, Hertfordshire, UK). The minireplicon pHH21-vMRen (SEQ ID NO:28) was also constructed.

For construction of pI.18_RVFV_L, the L segment coding region was assembled in a stepwise manner from four overlapping cDNA fragments. These fragments were generated by PCR using primer pairs RVFV_Sapl_L1for/RVFV_L1rev, RVFV_L2for/RVFV_L2rev, RVFV_L3for/RVFV_L3rev, and RVFV_L4for/RVFV_L4rev_SapI. The PCR fragments were individually TA-cloned into the pcDNA3.1-TOPO Vector (Invitrogen), giving rise to plasmids pcDNA3.1_RVFV_L1, pcDNA3.1_RVFV_L2, pcDNA3.1_RVFV_L3, and pcDNA3.1_RVFV_L4. Then, the insert of pcDNA3.1_RVFV_L2 was cut out with EcoRl and *Xho*I and cloned 3' of the L1 insert into the *Eco*RI/*Xho*I-digested pcDNA3.1_RVFV_L1. The resulting plasmid was named pcDNA3.1_RVFV_L1+L2.

In parallel, fragment L4 was subcloned into pI.18 using the *Bam*HI and Xhol restriction sites. The resulting plasmid, pl.18-L4 was then digested with *Sac*I and *Eco*RI. The gel-purified L4 fragment flanked by *Sac*I/*Eco*RI restriction sites at the 5' and 3' end, respectively, was then subcloned together with the *Bam*HI/*Sac*I-digested L3 fragment (cut out of pcDNA3.1_RVFV_L3) into the *Bam*HI/*Eco*RI-digested pI.18 vector. The resulting construct, pI.18_L3+L4, was then reopened with BamHl and Apal and the joined with the L1+L2 fragment cut out of pcDNA3.1_RVFV_L1+L2, again using *Bam*HI/*Apa*I. The amino acid sequence of the full-length insert in pI.18-RVFV L corresponds to the database entry DQ375403.

For construction of pI.18_RVFV_M two overlapping fragments were generated using primer pairs RVFV_SapI_m1for/RVFV_m1rev and RVFV_m2for/RVFV_m2rev_SapI. The two fragments were TA-cloned to generate plasmids pcDNA3.1_RVFV_M1 and pcDNA3.1_RVFV_M2, respectively. To obtain pI.18_RVFV_M, both fragments were finally cloned into the pI.18 vector in a three-way ligation reaction, where an internal *DraIII*-site served for joining of M1 and M2, and *Kpnl* and *Xhol* restriction sites were used for ligation into pI.18. The coding sequence of the insert corresponds to the database entry DQ380206, with the difference of one amino-acid substitution from leucine to glutamine at position 232.

Plasmid pI.18_RVFV_N was constructed by amplification of first-strand cDNA using PCR primers RVFV_SapI_Sfor and RVFV_C13_Srev_SapI. Subcloning of the resulting fragment into the TOPO vector first gave rise to plasmid pcDNA3.1_RVFV_N, from which the fragment was then cloned into the pI.18 vector using *KpnI*/*XhoI* sites. The sequence of the insert corresponds to database entry DQ380151.

The reporter plasmid pHH21_RVFV_vMRen contains the *Renilla* luciferase gene (Ren-Luc) in antisense orientation, flanked by the 3' and 5' genomic untranslated regions (UTRs) of the RVFV M segment. This reporter plasmid was generated in a three-step manner. First, the M UTRs seperated by a linker sequence containing two flanking *SapI* restriction sites and one central *KpnI* site were constructed from overlapping oligonucleotides RVFV_cMPro1for, RVFV_cMPro2rev, RVFV_cMPro3for, RVFV_cMPro4rev, RVFV_cMPro5for, RVFV_cMPro6rev as described previously (XXX). The resulting PCR fragment was TA-cloned to obtain pcDNA3.1_RVFV_MPro. Then, the UTR sequences including the linker were reamplified using primer pair RVFV_vMPro_pHH21for/ RVFV_vMPro_pHH21rev and cloned into the pHH21 backbone vector via primer-encoded *Esp3I* sites. This gave rise to precursor plasmid pHH21_RVFV_vMPro. In a last step, the Ren-Luc gene was amplified from plasmid pRL-SV40 (Promega) using primers RVFV_MRen_SapIfor and RVFV_MRen_SapIrev, and inserted into pHH21_RVFV_vMPro using *SapI* restriction sites, resulting in plasmid pHH21_RVFV_vMRen.

Plasmid pGL3-control (Promega) expresses firefly luciferase (FF-Luc) under control of a mammalian RNA polymerase II promoter.

Plasmid constructs encoding human MxA and the GTPase-deficient mutant T103A have been described previously [Ponten et al., J. of Virology 71:2591-9 (1997)]. For improved expression in mammalian cells we cloned the corresponding coding sequences into pI.18 via BamHI/EcoRI restriction sites, giving rise to plasmids pI.18-MxA and pI.18-HA-MxA(T103A).

### Example 2: RVFV Minireplicon system

Geneticin G418 (Biochrom AG) was dissolved in H₂O to 100mg/ml and used at a concentration of 1 mg/ml cell culture medium. BSR-T7/5 cells, Vero cells and 293T cells were cultivated in Dulbecco's modified Eagle's medium (DMEM) supplemented with 10% FCS.

Subconfluent monolayers of 293T cells in 12-well plates were transfected with 0.4 µg each pl.18_RVFV_N and pl.18_RVFV_L, 0.25µg pHH21_RVFV_vMRen, and 0.1µg pGL3-control using 3.5 µl Fugene HD transfection reagent (Roche) in 100 µl serum free medium (OptiMEM; Invitrogen). In some experiments, specific expression plasmids were omitted from the transfection mix. After transfection, cells were incubated for the indicated times and lysed in 100 µl Dual Luciferase Passive Lysis Buffer (Promega). An aliquot of 20 µl of the lysate was assayed for FF-Luc and Ren-Luc activities as described by the manufacturer (Promega).

Minireplicon systems are useful tools to investigate viral polymerase activity and promoter sequences. For bunyaviruses, they usually consist of a reporter gene flanked by viral 5' and 3' UTRs, and recombinant L and N proteins. The reporter construct is transfected into mammalian cells along with the L and N expression constructs and will be transcribed intracellularly into a minireplicon resembling a viral gene segment. The products of the expression constructs faithfully encapsidate, transcribe and replicate the minireplicon, thus resulting in reporter activity. For RVFV, minireplicon systems have been established which are based on the coexpressed RNA polymerase of the bacteriophage T7.

We disclose a T7-independent version in which the expression constructs were under control of a promoter for the cellular RNA polymerase II (RNAP II), and the minireplicon was transcribed intracellularly by RNAP I. The L and N genes were cloned into the high expression vector pl.18, and a minireplicon consisting of the antisense gene for the *Renilla* Luciferase (Ren-Luc) flanked by RVFV M segment UTRs was cloned into the RNAP I vector pHH21. Human 293T cells were transfected with these plasmids, and minireplicon activity was assessed after overnight incubation by measuring Ren-Luc activity of cell extracts.

Fig. 1 shows that coexpression of L, N and the minireplicon strongly upregulated the Ren-Luc activity (black bars), whereas omission of the L construct resulted in low activity. As the minireplicon plasmid was designed to produce negative sense RNA, a strong Ren-Luc activity clearly indicates successful packaging into RNPs and transcription by L and N. Moreover, the internal control consisting of the RNAP II-driven gene for the firefly luciferase (FF-Luc) was independent of the full set of RVFV plasmids (grey bars), as expected. Thus, our RVFV minireplicon system which is entirely based on the activity of cellular RNA polymerases was able to efficiently reconstitute recombinant RVFV RNPs.

### Example 3: Preparation of virus-like particles

Subconfluent monolayers of 293T cells in 6-well plates (donor cells) were transfected with 0.5µg of plasmids pI.18_RVFV_N, pI.18_RVFV_L, pI.18_RVFV_M, pHH21_RVFV_vMRen, and with 0.1µg of pGL3-control using 6.3µl Fugene HD transfection reagent. At the indicated timepoints supernatants were harvested and donor cells were lysed with 200µl Passive Lysis Buffer to measure luciferase activities as described above. The supernatants were frozen for at least one hour at -20°C, treated with 1 µl/ml Benzonase (Novagen) at 37°C for three hours, and centrifuged at 12.000 × g for 5 min to remove cellular debris. Aliquots of the treated supernatants were then used to infect infect new cells (indicator cells). In some experiments, indicator cells were transfected with L and N expression plasmids 16 h prior to the transfer of supernatant to support replication and transcription of RNPs.

For two bunyaviruses, Bunyamwera virus and Uukuniemi virus, VLP systems consisting of minireplicon RNPs packaged by viral glycoproteins were established and have been proven useful for the analysis of particle assembly and RNP packaging. To achieve this for RVFV, we cloned the reading frame of the viral M segment (encoding Gn, Gc, NSm, and the 78kD protein) into the RNAP II expression vector pI.18 (see Example 1). Immunofluorescense analyses using specific antisera confirmed expression of RVFV glycoproteins and their transport to the plasma membrane.

To generate VLPs, the M expression construct was cotransfected with the minireplicon system plasmids into 293T cells (donor cells). Twenty-four hours later, supernatants were harvested and used to infect BSR-T7/5 cells expressing RVFV L and N (indicator cells). An outline of the experiment is provided in Fig. 2 A.

Two additional measures were taken to minimize unspecific reporter activities in indicator cells caused by carryover transfection. Firstly, any residual plasmids were destroyed by extensive treatment of VLP supernatants with DNase. Secondly, the species-specificity of the human RNAP I-driven minireplicon construct excluded background expression in the non-human indicator cells. Moreover, RNAP II-driven coexpression of FF-Luc in donor cells served as a specificity control, because the FF-Luc RNA lacks any RVFV sequences and should therefore not be packaged into VLPs.

Reporter assays shown in Fig 2 B (left panel) demonstrate again the strong minireplicon activity mediated by L and N. Coexpression of the M segment-encoded glycoproteins had no detrimental effects.

Importantly, after transfer of supernatants to indicator cells, only the sample which had received the M expression construct in addition to the minireplicon system displayed a strong Ren-Luc activity. FF-Luc activity, by contrast, was neglectable in indicator cells. These data implicate that coexpression of the M segment-encoded genes results in efficient and specific packaging of minireplicon RNPs into VLPs, which are subsequently released into the medium, able to infect new cells.

We further analysed VLP characteristics by using neutralising antisera. VLP-containing supernatants were first incubated with different antisera derived from RVFV-infected mice or humans, and then used to infect indicator cells expressing RVFV L and N.

Proteins were separated by SDS-PAGE and transferred to a PVDF membrane (Amersham), followed by incubation in saturation buffer (PBS containing 10% non-fat dry milk and 0.05% Tween). The membrane was first incubated for one hour with primary antibodies, washed three times with 0.05% PBS-Tween, followed by incubation with a horseradish-peroxidase (HRP)-conjugated secondary antibody. After three additional washing steps, detection was performed using the SuperSignal West Femto chemiluminescence kit (Pierce).

Fig. 3A shows that a strong reduction in Ren-Luc activity occurs in receptor cells when RVFV-specific antisera were used. By contrast, a control antibody directed against the La Crosse virus envelope protein as well as sera from an uninfected mouse or individual had no neutralising activity.

The protein composition of VLPs was determined. To this aim, supernatants from 293T cells transfected with different plasmid combinations were purified and subjected to Western blot analysis (Fig. 3B). Supernatants from RVFV-infected cells served as positive control (lane 1). As expected, there was no release of viral proteins from minireplicon-expressing cells lacking glycoprotein expression (lane 3). In contrast, both the Gn and N protein could be detected in supernatants where the full set of plasmids for the VLP system had been transfected (lane 2), indicating that the composition of VLPs is similar to that of RVFV particles. We noted, however, that the relative proportion of the 78kD protein (which is recognized by the monoclonal antibody against Gn) was substantially higher in VLPs when compared to virus particles. Apparently, expression or particle incorporation of the M segment-ecoded proteins differs between plasmid-transfected and virus-infected cells. Together, these data indicate that the VLPs generated by coexpression of the minireplicon system and the M-encoded glycoproteins can serve not only as a model for authentic RVFV particles, but also for immunization of humans.

### Example 4 Concentration and optimization of RVF virus-like particles

### a) Concentration

Supernatants from VLP-expressing cells were collected and clarified from cell debris by centrifugation (12.000 × g, 10 min at 4°C). The particles were then concentrated by ultracentrifugation at 24,000 rpm and 4°C for 2 h in a Beckman SW41Ti rotor. The pellet was dried for 5 min before resuspension in phosphate-buffered saline (PBS).

Concentrated VLPs were used in the Western Blot shown in Fig. 3B and for vaccination.

### b) Optimization of VLP production

A time course analysis was performed to study the kinetics of VLP formation. Donor cells were transfected with either the minireplicon alone, or in addition with the M-expression construct to generate VLPs. From parallel dishes, cell lysates and supernatants were harvested after 24, 48 or 72 hours. Reporter assays of donor cell lysates showed again (compare Fig. 2 B, left panel) that addition of the M segment expression plasmid had no substantial effect on minireplicon activity (Fig. 4A, columns 2 and 3). Both the minireplicon and the VLP donor cells displayed peak activity at 48 h post-transcription, and a decrease at the 72 h time point.

This indicates robust and continued production of recombinant RVFV components and, most probably, exhaustion after longer expression times. When supernatants of the time course were used to infect indicator cells, reporter assays confirmed again that the M segment expression plasmid was necessary to generate infectious VLPs (Fig. 4B). Moreover, VLP activities steadily increased in the supernatants harvested from 24 h to 72 h after transfection of donor cells. The apparent discrepancy between donor cells (peak at 48 h time point) and indicator cells (steady increase until 72 h) may simply be due to the fact that VLPs are relatively stable and accumulate in the supernatants over the whole time course, whereas gene expression in donor cells fades out.

Therefore, defined conditions for the highly efficient production of VLPs are disclosed.

### Example 5: Primary transcription of genes packaged in VLPs

Conventionally, primary transcription of negative-strand RNA viruses is measured by using translational inhibitors, e.g. cycloheximide. Cutting off the supply of newly synthesized viral proteins allows to measure the activity of incoming polymerase complexes without the amplification loop established by genome replication. However, the fact that bunyavirus mRNA transcription is strictly dependent on concurrent translation precludes a dissection of primary and secondary transcription by pharmaceutical means.

It was checked whether the VLP system would allow the measurement of RVFV primary transcription. Thus, BSR-T7/5 cells were infected with VLPs and their transcriptional activity was monitored by measuring Ren-Luc over a period of 48 h. To distinguish between primary and secondary transcription, the indicator cells were either left untransfected or were transfected with polymerase complex proteins N and L prior to infection (Fig. 5). In parallel, supernatants from minireplicon-expressing cells were used to detect potential background reporter activity. As expected, strong Ren-Luc activity was observed in VLP-infected cells where polymerase complexes were present (Fig. 5A). This activity steadily increased until 48 h post-infection, the last timepoint measured, thus being indicative for continuous replication and transcription. Supernatants from minireplicon-only transfected cells, by contrast, did not elicit any detectable Ren-Luc activity. Interestingly, a clear and reproducible Ren-Luc activity over background was also measured in VLP-infected naïve (untransfected) cells, albeit to a much lower extent than in N and L-expressing cells. Maximum activity was reached after 24 h and remained at an almost constant level for further 24 h. This indicates that the VLP-associated viral polymerase is highly active in primary transcription. The same observation was also made in several other cell lines infected with VLPs. The established system thus allows, for the first time, to study primary transcription of a bunyavirus.

### Example 6: Effect of the antiviral protein MxA on VLP transcription

The VLP system of the present application offers the chance to dissect the effect of the antivirally active protein MxA on primary and secondary transcription of bunyaviruses. To investigate this, indicator cells were transfected with an expression construct for MxA and infected with RVFV VLPs. The MxA mutant T103A served as a negative control. Fig. 6A (upper part) shows that MxA expressed by indicator cells was capable of reducing the reporter activity of incoming VLPs, indicating an effect on primary transcription. Moreover, if N and L support constructs were expressed in addition to MxA, a similar, but somewhat stronger inhibitory effect was observed, although the VLP reporter activity was much higher as compared to the primary transcription set-up (Fig. 6B, upper part). Western blot analyses for MxA and RVFV N were performed to control recombinant gene expression (Fig. 6A and B, lower parts). These data suggest that MxA is capable of interfering with primary transcription of bunyaviruses and hence interacts not only with the soluble N protein, but also with assembled RNPs.

In summary, reverse genetics tools have been established which enable the study of RVFV RNP activity, virus assembly, virus entrance, primary transcription and antiviral mechanisms under non-BSL-3 conditions. Moreover, the modular buildup of those systems allows to dissect the viral life cycle in a systematic manner.

### Example 7: Vaccination of mice

VLPs according to the teaching of the present application whereby the VLPs contained the gene coding for N were produced and concentrated as described in Example 4a).

Two groups of six mice each (C57/BL6) were immunized intraperitoneally with either PBS (as control) or with 1,000,000 VLPs.

Two weeks after the immunization both groups of mice were challenged with 100,000 pfu (plaque-forming units) of the Rift Valley Fever Virus strain ZH548 intraperitoneally.

In the control group (PBS) five of six mice died.

From the group of mice immunized with VLPs according to the present invention all six mice survived the viral challenge.

It is very surprising that after a single vaccination a protection could be obtained after 14 days. Alternatively it is possible to repeat the immunization in such a manner that the animal is immunized two or three times after suitable periods of time ranging between two weeks and three months.

## Claims

1. A method for producing virus-like particles comprising
a) transfecting a mammalian cell line with several independent vectors comprising
aa) a vector containing at least a substantial part of the M gene from Rift Valley Fever Virus,
bb) a vector containing at least a substantial part of the L gene from Rift Valley Fever Virus,
cc) a vector containing at least a substantial part of the N gene from Rift Valley Fever Virus and
dd) a vector containing a multicloning site flanked by the non-coding 5'-and 3'-ends of the M-segment of Rift Valley Fever Virus, and
b) culturing the transfected mammalian cell line under suitable conditions and obtaining the VLPs from the supernatant of the transfected cultured cell lines.

2. Method according to claim 1 wherein the mammalian cell line is a human cell line.

3. Method according to claim 1 wherein the vector containing the non-coding 5'- and 3'-ends of the M segment of the Rift Valley Fever Virus and a multicloning site contains an additional gene inserted within the multicloning site.

4. Method according to claim 3 wherein the gene coding for the luciferase obtained from *Renilla* is inserted within the multicloning site.

5. Method according to claim 3 wherein the gene coding for the N protein of Rift Valley Fever Virus has been inserted within the multicloning site.

6. Method according to claim 3 wherein the gene coding for the L protein of Rift Valley Fever Virus has been inserted within the multicloning site.

7. Virus-like particle comprising at least one protein of the Rift Valley Fever Virus obtainable by a method according to any of claims 1 to 6.

8. Pharmaceutical composition comprising a virus-like particle according to claim 7.

9. Use of virus-like particle according to claim 7 for the vaccination of humans against an infection of Rift Valley Fever Virus.

10. Use of virus-like particle according to claim 7 for the vaccination of animals against an infection of Rift Valley Fever Virus.

11. Method of testing an agent for antiviral activity against Rift Valley Fever Virus wherein
a) virus-like particles according to claim 7 are brought into contact with a mammalian cell line and the agent to be tested for antiviral activity and
b) the antiviral activity is measured by comparison with a control wherein the agent to be tested is replaced by a suitable control which does not have antiviral activity

12. Method according to claim 11 wherein the agent to be tested is selected from the group comprising anti-RVFV antibodies or fragments thereof, chemically synthesized compounds supposed to have antiviral activity or proteins, peptides, steroids of natural origin and derivatives thereof supposed to have antiviral activity against Rift Valley Fever Virus.

13. Testkit for performing a method according to claims 11 or 12, **characterized in that** the testkit comprises a mammalian cell line and virus-like particles according to claim 7.


SEQUENCE LISTING

<110>  UniversitätsKlinikum Freiburg

<120>  Rift Valley Fever Virus - like Particles and its use for
       immunisation

<130>  ZEE20071108

<160>  28

<170>  PatentIn version 3.5

<210>  1
<211>  43
<212>  DNA
<213>  artificial sequence

<220>
<223>  primer

<400>  1
gacagagctc ttcatcatgg attctatatt atcaaaacag ctg                        43


<210>  2
<211>  18
<212>  DNA
<213>  artificial sequence

<220>
<223>  primer

<400>  2
tgaggcatcc attgctgc                                                    18


<210>  3
<211>  19
<212>  DNA
<213>  artificial sequence

<220>
<223>  primer

<400>  3
gctgggcctt tgatctctc                                                   19


<210>  4
<211>  18
<212>  DNA
<213>  artificial sequence

<220>
<223>  primer

<400>  4
ctcccgatga ccatccag                                                    18


<210>  5
<211>  22
<212>  DNA
<213>  artificial sequence

<220>
<223>  primer

<400>  5
gaggaaagaa ttgttcaatc gg                                                22

<210>  6
<211>  22
<212>  DNA
<213>  artificial sequence

<220>
<223>  primer

<400>  6
aatggtcacg gataaccata gc                                                22

<210>  7
<211>  22
<212>  DNA
<213>  artificial sequence

<220>
<223>  primer

<400>  7
aagcaactct agctcacacc cc                                                22

<210>  8
<211>  36
<212>  DNA
<213>  artificial sequence

<220>
<223>  primer

<400>  8
gacagagctc ttctggtctt agcctagcat gtcatc                                 36

<210>  9
<211>  46
<212>  DNA
<213>  artificial sequence

<220>
<223>  primer

<400>  9
gacagagctc ttctaaatgt atgttttatt aacaattcta atctcg                      46

<210>  10
<211>  18
<212>  DNA
<213>  artificial sequence

<220>
<223>  primer

<400>  10
tcaaacaagc ctctgccc                                                     18

<210>  11
<211>  21
<212>  DNA

<213>  artificial sequence

<220>
<223>  primer

<400>  11
gtgaacaggg aaataggatg g                                                21


<210>  12
<211>  37
<212>  DNA
<213>  artificial sequence

<220>
<223>  primer

<400>  12
gacagagctc ttcctgatct atgaggcctt cttagtg                               37


<210>  13
<211>  39
<212>  DNA
<213>  artrificial sequence

<400>  13
gacagagctc ttcataatgg acaactatca agagcttgc                             39


<210>  14
<211>  36
<212>  DNA
<213>  artificial sequence

<220>
<223>  primer

<400>  14
gacagagctc ttccaagcag caaaagagga catttc                                36


<210>  15
<211>  80
<212>  DNA
<213>  artificial sequence

<220>
<223>  primer

<400>  15
gacagacgtc tcctatagac acaaagacgg tgcattaaat gaagagcggt accgctcttc      60

atcagtacgt gtaaaagcaa                                                  80


<210>  16
<211>  80
<212>  DNA
<213>  artificial sequence

<220>
<223>  primer

<400>  16
tgacctatct gtacaatact tacataatta ggtttgctta tgtctactta tttcaacata      60

ttgcttttac acgtactgat                                                  80

<210> 17
<211> 80
<212> DNA
<213> artificial sequence

<220>
<223> primer

<400> 17
agtattgtac agataggtca aattattgga atatccaagc ttagaaactt atgcaataat 60

actttagatg taagcttagt 80


<210> 18
<211> 80
<212> DNA
<213> artificial sequence

<220>
<223> primer

<400> 18
actagaatat tcacaagcac ttgagactgc tgctgcctca ccccaccacc ccaaattaca 60

actaagctta catctaaagt 80


<210> 19
<211> 80
<212> DNA
<213> artificial sequence

<220>
<223> primer

<400> 19
gtgcttgtga atattctagt tggcgtaatc gtcttttgcc agattagctg ggaattaaac 60

taactctttg aagttgcacc 80


<210> 20
<211> 48
<212> DNA
<213> artificial sequence

<220>
<223> primer

<400> 20
tctgtccgtc tccacccaca caaagaccgg tgcaacttca aagagtta 48


<210> 21
<211> 39
<212> DNA
<213> artificial sequence

<220>
<223> primer

<400> 21
gacagacgtc tcctattaca caaagaccgg tgcaacttc 39

<210> 22
<211> 36
<212> DNA
<213> artificial sequence

<220>
<223> primer

<400> 22
gacagacgtc tccgggacac aaagacggtg cattaa 36


<210> 23
<211> 41
<212> DNA
<213> artificial sequence

<220>
<223> primer

<400> 23
gacagagctc ttcaaaatga cttcgaaagt ttatgatcca g 41


<210> 24
<211> 43
<212> DNA
<213> artificial sequence

<220>
<223> primer

<400> 24
gacagagctc ttcttgactt attgttcatt tttgagaact cgc 43


<210> 25
<211> 10608
<212> DNA
<213> artficial sequence

<400> 25
gtggcctaac tacggctaca ctagaagaac agtatttggt atctgcgctc tgctgaagcc 60

agttaccttc ggaaaaagag ttggtagctc ttgatccggc aaacaaacca ccgctggtag 120

cggtggtttt tttgtttgca agcagcagat tacgcgcaga aaaaaaggat ctcaagaaga 180

tcctttgatc ttttctacgg ggtctgacgc tcagtggaac gaaaactcac gttaagggat 240

tttggtcatg agattatcaa aaaggatctt cacctagatc cttttaaatt aaaaatgaag 300

ttttaaatca atctaaagta tatatgagta aacttggtct gacagttacc aatgcttaat 360

cagtgaggca cctatctcag cgatctgtct atttcgttca tccatagttg cctgactccc 420

cgtcgtgtag ataactacga tacgggaggg cttaccatct ggccccagtg ctgcaatgat 480

accgcgagac ccacgctcac cggctccaga tttatcagca ataaaccagc cagccggaag 540

ggccgagcgc agaagtggtc ctgcaacttt atccgcctcc atccagtcta ttaattgttg 600

ccgggaagct agagtaagta gttcgccagt taatagtttg cgcaacgttg ttgccattgc 660

tacaggcatc gtggtgtcac gctcgtcgtt tggtatggct tcattcagct ccggttccca 720

acgatcaagg cgagttacat gatcccccat gttgtgcaaa aaagcggtta gctccttcgg 780

tcctccgatc gttgtcagaa gtaagttggc cgcagtgtta tcactcatgg ttatggcagc 840
actgcataat tctcttactg tcatgccatc cgtaagatgc ttttctgtga ctggtgagta 900
ctcaaccaag tcattctgag aatagtgtat gcggcgaccg agttgctctt gcccggcgtc 960
aatacgggat aataccgcgc cacatagcag aactttaaaa gtgctcatca ttggaaaacg 1020
ttcttcgggg cgaaaactct caaggatctt accgctgttg agatccagtt cgatgtaacc 1080
cactcgtgca cccaactgat cttcagcatc ttttactttc accagcgttt ctgggtgagc 1140
aaaaacagga aggcaaaatg ccgcaaaaaa gggaataagg gcgacacgga aatgttgaat 1200
actcatactc ttcctttttc aatattattg aagcatttat cagggttatt gtctcatgag 1260
cggatacata tttgaatgta tttagaaaaa taaacaaata ggggttccgc gcacatttcc 1320
ccgaaaagtg ccacctgacg tctaagaaac cattattatc atgacattaa cctataaaaa 1380
taggcgtatc acgaggccct ttcgtctcgc gcgtttcggt gatgacggtg aaaacctctg 1440
acacatgcag ctcccggaga cggtcacagc ttgtctgtaa gcggatgccg ggagcagaca 1500
agcccgtcag ggcgcgtcag cgggtgttgg cgggtgtcgg ggctggctta actatgcggc 1560
atcagagcag attgtactga gagtgcacca tatgcggtgt gaaataccgc acagatgcgt 1620
aaggagaaaa taccgcatca ggcgccattc gccattcagg ctgcgcaact gttgggaagg 1680
gcgatcggtg cgggcctctt cgctattacg ccagctggcg aaagggggat gtgctgcaag 1740
gcgattaagt tgggtaacgc cagggttttc ccagtcacga cgttgtaaaa cgacggccag 1800
tgccaagctt gcatgcctgc aggtcaattc cctggcatta tgcccagtac atgaccttat 1860
gggactttcc tacttggcag tacatctacg tattagtcat cgctattacc atggtgatgc 1920
ggttttggca gtacatcaat gggcgtggat agcggtttga ctcacgggga tttccaagtc 1980
tccaccccat tgacgtcaat gggagtttgt tttggcacca aaatcaacgg gactttccaa 2040
aatgtcgtaa caactccgcc ccattgacgc aaatgggcgg taggcgtgta cggtgggagg 2100
tctatataag cagagctcgt ttagtgaacc gtcagatcgc ctggagacgc catccacgct 2160
gttttgacct ccatagaaga caccgggacc gatccagcct ggggatctag cctccgcggc 2220
cgggaacggt gcattggaac gcggattccc cgtgccaaga gtgacgtaag taccgcctat 2280
agagtctata ggcccacccc cttggcttct tatgcatgct atactgtttt tggcttgggg 2340
tctatacacc cccgcttcct catgttatag gtgatggtat agcttagcct ataggtgtgg 2400
gttattgacc attattgacc actcccctat tggtgacgat ctttccatta ctaatccata 2460
acatggctct ttgccacaac tctctttatt ggctatatgc caatacactg tccttcagag 2520
actgacacgg actctgtatt tttacaggat ggggtctcat ttattattta caaattcaca 2580
tatacaacac caccgtcccc agtgcccgca gtttttatta aacataacgt gggatctcca 2640
cgcgaatctc gggtacgtgt tccggacatg ggctcttctc cggtagcggc ggagcttcta 2700
catccgagcc ctgctcccat gcctccagcg actcatggtc gctcggcagc tccttgctcc 2760
taacagtgga ggccagactt aggcacagca cgatgcccac caccaccagt gtgccgcaca 2820

aggccgtggc ggtagggtat gtgtctgaaa atgagctcgg ggagcgggct tgcaccgctg 2880
acgcatttgg aagacttaag gcagcggcag aagaagatgc aggcagctga gttgttgtgt 2940
tctgataaga gtcagaggta actcccgttg cggtgctgtt aacggtggag ggcagtgtag 3000
tctgagcagt actcgttgct gccgcgcgcg ccaccagaca taatagctga cagactaaca 3060
gactgttcct ttccatgggt cttttctgca gtcaccgtcc ttgacacgat cggatcccgg 3120
gtaccgagct cggatccagt acccttcacc gacagagctc ttcatcatgg attctatatt 3180
atcaaaacag ctggttgaca agactggttt tgttagagtg ccaatcaagc attttgactg 3240
tacaatgcta actctggcac ttccaacatt tgatgtttcc aagatggtag atagaattac 3300
catagacttc aatctggatg atatacaagg agcatctgaa ataggctcaa ctttgctacc 3360
ctccatgtcg atagatgtgg aagatatggc caattttgtt cacgatttca cctttggcca 3420
cttagctgac aagactgaca gactgttaat gcgtgagttt cccatgatga atgacgggtt 3480
tgatcatttg agccctgaca tgatcattaa aactacatct ggcatgtaca acatcgttga 3540
gttcaccacc tttaggggag atgaaagagg tgcattccag gctgccatga ctaaactcgc 3600
taagtatgag gttccttgtg agaacagatc tcagggcagg actgttgttc tttatgttgt 3660
tagtgcttat cggcatggtg tatggtctaa tctggagcta gaggactctg aagcagagga 3720
gatggtttat aggtacagac ttgctcttag tgtgatggat gagctaagga ccttgttccc 3780
agaactgtca tccacagatg aggaactagg gaagactgag agagagttgc tagccatggt 3840
ctcctccatc caaataaatt ggtcagtcac agaatctgtg tttccaccct tcagcagaga 3900
aatgtttgac aggtttagat cctcccctcc cgattcagag tatatcacga ggatagtgag 3960
cagatgccta ataaattctc aagagaaact catcaatagt tccttctttg ctgaagggaa 4020
tgataaggct ctgagatttt caaaaaacgc tgaagagtgt tccttggcag tagagagagc 4080
cttaaatcag tatagagcag aagacaacct tagggacctc aatgaccaca agtcaactat 4140
tcagctgcct ccctggctgt cctatcatga tgtcgatggc aaagatctgt gccctcttca 4200
gggactagat gtgagagggg accatcccat gtgcaacttg tggagggaag tggtcacctc 4260
tgcaaaccta gaggagattg agaggatgca cgatgatgca gcagcagaac ttgagtttgc 4320
cctttcggga gtaaaggaca ggccagatga gagaaacaga taccatagag tccacctaaa 4380
tatgggctca gatgatagtg tctacatagc tgctttagga gttaatggaa agaagcataa 4440
agcagacact ttagtgcaac aaatgagaga caggagtaaa cagcctttct ccccagacca 4500
cgatgtggat cacatatctg aatttctctc tgcatgctct agtgacttgt gggcaacaga 4560
tgaggacctg tacagccctc tctcttgtga taaagagctt agattggcag cccagaggat 4620
tcatcagcca tccttgtcag aaaggggttt caatgagatc ataacagagc actacaaatt 4680
catgggaagt aggataggtt catggtgcca aatggtcagc ttgataggag ctgagctatc 4740
agcttctgtt aaacaacatg tcaagcctaa ctactttgtg attaaacgac tactaggttc 4800
tgggattttc ttgctaatca agcccacttc cagcaaaagc catatatttg tgtcttttgc 4860

aattaagcgc tcttgctggg cctttgatct ctccacttcc agggttttca agccctacat 4920
agatgctggg gatctgttag ttactgactt tgtttcttat aagctaagca agcttaccaa 4980
cctctgcaag tgcgtttcat taatggagtc ctccttctca ttctgggcag aagcatttgg 5040
aattccaagc tggaactttg ttggtgactt gttcaggtct tcagactctg cagcaatgga 5100
tgcctcatac atgggcaaac tttctttatt aacccttttg gaagacaaag cagcaactga 5160
agagttacag actattgcaa gatatataat catggagggc tttgtctcgc ccccagaaat 5220
cccaaaacct cacaagatga cctctaagtt tcctaaggtt ctcaggtcag agctgcaggt 5280
ttacttatta aactgcttat gcagaactat ccagagaata gcaggtgagc ccttcattct 5340
taagaagaag gatgggtcta tatcctgggg tggcatgttc aatccttttt cagggcgtcc 5400
actgcttgat atgcaaccac tcatcagctg ttgttacaat ggttacttta aaaataaaga 5460
agaagagact gagccttcgt ccctttctgg gatgtataag aaaatcatag aacttgagca 5520
ccttagacca cagtcagatg ccttcttggg ttacaaagat ccagaacttc ccagaatgca 5580
tgagttcagt gtttcctact tgaaggaggc ttgcaatcat gctaagctag tcttgaggag 5640
cctctatgga cagaatttca tggagcagat agacaaccag attattcgag agctcagtgg 5700
gttgactcta gaaaggttgg ccacacttaa ggccacaagc aactttaatg agaattggta 5760
tgtctataag gatgtagcag acaaaaacta cacaagggat aaattattag tgaagatgtc 5820
aaaatatgcc tctgagggaa agagcctagc tatccagaag tttgaggatt gtatgaggca 5880
gatagagtca caaggatgca tgcatatttg tttgtttaag aaacaacagc atggaggtct 5940
gagagagatc tatgtgatgg gtgcagagga aagaattgtt caatcggtgg tggagacaat 6000
agccaggtcc atagggaagt tctttgcttc tgataccctc tgtaaccccc ccaataaagt 6060
gaaaattcct gagacacatg gcatcagggc ccggaagcaa tgtaaggggc ctgtgtggac 6120
ttgtgcaaca tcagatgatg caaggaagtg gaaccaaggc cattttgtta caaagtttgc 6180
cctcatgctg tgtgagttca cctctcctaa atggtggccg ctgatcatta ggggatgctc 6240
aatgtttacc aggaaaagga tgatgatgaa tttgaattat cttaagatcc tggatggtca 6300
tcgggagctt gatattagag atgactttgt aatggatctc ttcaaagctt atcatggcga 6360
ggcagaagtt ccatgggcct ttaaaggcaa aacatatttg gaaaccacaa cagggatgat 6420
gcagggaata ctgcattata cttcctcact attacacacc attcaccaag aatacatccg 6480
gtccttgtcc tttaagatat tcaacctgaa ggttgctcct gagatgagca agagcctggt 6540
ttgtgacatg atgcaaggat cagatgatag tagtatgcta atcagcttcc cagctgatga 6600
tgagaaggtt cttaccagat gcaaagtggc cgcagctata tgcttccgca tgaagaagga 6660
gctgggagtg taccttgcca tttacccctc agagaagtcc acagcaaaca cagattttgt 6720
gatggagtac aattctgaat tttatttcca cacccagcat gttagaccaa cgatcaggtg 6780
gattgcagct tgttgcagcc tgccagaagt ggaaacacta gtagcccgcc aggaagaggc 6840
ctctaaccta atgacttcag ttactgaggg aggtgggtca ttctccttag ctgcaatgat 6900

tcagcaagct cagtgtactc tccattacat gctgatgggc atgggagtgt ctgagctatt 6960
cttagagtat aagaaggcag tgctgaagtg gaatgaccct ggcctgggtt tcttcctgct 7020
tgacaatcct tatgcgtgcg gattgggagg tttcagattt aatctcttca aagctatcac 7080
cagaactgat ttgcagaagc tatatgcttt cttcatgaag aaggtcaagg gctcagctgc 7140
tagggactgg gcagatgaag atgtcaccat cccagaaacg tgtagcgtga gcccaggtgg 7200
cgctctaatt cttagctcct ctctaaagtg gggatctagg aagaagtttc agaaattgag 7260
agaccgtttg aacataccag agaactggat tgaactaata aatgagaatc cagaggtgct 7320
ctatcgggct cccagaacag gcccagaaat attgttgcgc attgcagaga aagtccatag 7380
cccaggtgtt gtgtcatcat tgtcttctgg caatgcagtt tgtaaagtca tggcctcagc 7440
tgtatacttc ttatcagcaa caatttttga ggacactgga cgtcctgagt tcaacttctt 7500
ggaggattct aagtacagct tgctacaaaa gatggctgca tattctggct ttcatggttt 7560
caatgatatg gagccagaag atatattatt cttattcccg aatattgagg aattagaatc 7620
actggattct atagtttaca acaagggaga aatagacatc atcccaagag tcaacatcag 7680
ggatgcaacc caaaccaggg tcactatctt taatgagcag aagaccctcc ggacatctcc 7740
agagaagttg gtgtcagaca agtggtttgg gactcagaag agtaggatag gcaaaacaac 7800
cttcctggct gaatgggaaa agctaaagaa aattgtaaag tggttggaag acactccaga 7860
agcaactcta gctcacaccc cactgaataa ccatattcaa gttaggaatt tctttgctag 7920
aatggaaagc aagcctagaa cagtcagaat aacaggagct ccagtaaaga agaggtcagg 7980
ggttagtaag atagctatgg ttatccgtga caatttctcc cggatgggcc atcttcgagg 8040
tgtagaagac cttgctggct tcactcgtag tgtgtcagct gaaattctca agcactttct 8100
attctgtata ctacaaggtc catacagtga gagctataag ctacagctaa tctacagagt 8160
cctaagctca gtgtcaaacg ttgagataaa ggaatcagat ggtaagacaa aaaccaactt 8220
gattggaatc cttcagagat ttctagatgg tgatcacgtt gtccccataa ttgaagagat 8280
gggagccgga acagtgggtg gattcatcaa gagacaacaa tctaaagttg tgcagaacaa 8340
agtggtctat tatggagttg ggatttggag aggcttcatg gatggatatc aggtccatct 8400
agagatagaa aatgacatag gacagccccc aaggcttagg aatgtcacaa ctaactgtca 8460
gagcagccca tgggacctga gtattccaat aaggcaatgg gcagaagaca tgggggtcac 8520
aaacaaccag gattattctt ctaaatctag caggggggcc agatattgga tgcattcatt 8580
caggatgcaa ggacctagca agccatttgg atgcccagtt tatattatta agggtgatat 8640
gtcagatgtc atcagactga gaaaggagga ggtggagatg aaagtacggg gctctactct 8700
caacttgtac accaagcacc attctcatca ggacctacac attctatctt acactgcatc 8760
agacaatgat ctcagtccag gcattttcaa gtcaatatca gatgaggggg tggctcaagc 8820
cctgcaatta tttgagaggg agccaagcaa ctgctgggtg agatgtgagt ctgtagcccc 8880
aaaatttata tcagccatcc ttgagatatg tgaggggaag agacagataa agggaattaa 8940

cagaaccaga ctctcagaga ttgtgagaat ttgttctgaa tcttccctaa gatcaaaagt 9000
cggatctatg ttctcatttg tcgccaatgt cgaggaggcc catgatgttg attatgatgc 9060
gttaatggat ctaatgatag aggatgccaa gaacaatgca ttcagtcatg ttgttgactg 9120
catagagttg gatgttagtg gcccttacga gatggagtct tttgatacat ctgatgtcaa 9180
tctctttggg ccagcccatt acaaggacat cagttcatta tctatgattg ctcatccctt 9240
aatggataag tttgttgatt atgctatttc taagatgggg agagcctcag ttaggaaagt 9300
tctagaaaca ggtcggtgct ccagcaaaga ctatgattta tcaaaggttc tcttcagaac 9360
tctacagaga ccagaagaaa gcattaggat agatgatctg gaattatatg aggagacaga 9420
tgtggcggat gacatgctag gctaagacca gaagagctct gtcaagggtc aagacaattc 9480
tgcagatatc cagcacagtg gcggccgctc gaggaattct agatcccacg tcactattgt 9540
atactctata ttatactcta tgttatactc tgtaatccta ctcaataaac gtgtcacgcc 9600
tgtgaaaccg tactaagtct cccgtgtctt cttatcacca tcaggtgaca tcctcgccca 9660
ggctgtcaat catgccggta tcgattccag tagcaccggc cccacgctga caacccactc 9720
ttgcagcgtt agcagcgccc ctcttaacaa gccgaccccc accagcgtcg cggttactaa 9780
cactcctctc cccgacctgc agcccaagct tggcgtaatc atggtcatag ctgtttcctg 9840
tgtgaaattg ttatccgctc acaattccac acaacatacg agccggaagc ataaagtgta 9900
aagcctgggg tgcctaatga gtgagctaac tcacattaat tgcgttgcgc tcactgcccg 9960
ctttccagtc gggaaacctg tcgtgccagc tgcattaatg aatcggccaa cgcgcgggga 10020
gaggcggttt gcgtattggg cgctcttccg cttcctcgct cactgactcg ctgcgctcgg 10080
tcgttcggct gcggcgagcg gtatcagctc actcaaaggc ggtaatacgg ttatccacag 10140
aatcagggga taacgcagga aagaacatgt gagcaaaagg ccagcaaaag gccaggaacc 10200
gtaaaaaggc cgcgttgctg gcgtttttcc ataggctccg cccccctgac gagcatcaca 10260
aaaatcgacg ctcaagtcag aggtggcgaa acccgacagg actataaaga taccaggcgt 10320
ttccccctgg aagctccctc gtgcgctctc ctgttccgac cctgccgctt accggatacc 10380
tgtccgcctt tctcccttcg ggaagcgtgg cgctttctca tagctcacgc tgtaggtatc 10440
tcagttcggt gtaggtcgtt cgctccaagc tgggctgtgt gcacgaaccc cccgttcagc 10500
ccgaccgctg cgccttatcc ggtaactatc gtcttgagtc caacccggta agacacgact 10560
tatcgccact ggcagcagcc actggtaaca ggattagcag agcgaggt 10608

<210> 26
<211> 7954
<212> DNA
<213> artificial sequence

<220>
<223> vector

<400> 26
atgtaggcgg tgctacagag ttcttgaagt ggtggcctaa ctacggctac actagaagaa 60

cagtatttgg tatctgcgct ctgctgaagc cagttacctt cggaaaaaga gttggtagct 120
cttgatccgg caaacaaacc accgctggta gcggtggttt ttttgtttgc aagcagcaga 180
ttacgcgcag aaaaaaagga tctcaagaag atcctttgat cttttctacg gggtctgacg 240
ctcagtggaa cgaaaactca cgttaaggga ttttggtcat gagattatca aaaaggatct 300
tcacctagat ccttttaaat taaaaatgaa gttttaaatc aatctaaagt atatatgagt 360
aaacttggtc tgacagttac caatgcttaa tcagtgaggc acctatctca gcgatctgtc 420
tatttcgttc atccatagtt gcctgactcc ccgtcgtgta gataactacg atacgggagg 480
gcttaccatc tggccccagt gctgcaatga taccgcgaga cccacgctca ccggctccag 540
atttatcagc aataaaccag ccagccggaa gggccgagcg cagaagtggt cctgcaactt 600
tatccgcctc catccagtct attaattgtt gccgggaagc tagagtaagt agttcgccag 660
ttaatagttt gcgcaacgtt gttgccattg ctacaggcat cgtggtgtca cgctcgtcgt 720
ttggtatggc ttcattcagc tccggttccc aacgatcaag gcgagttaca tgatccccca 780
tgttgtgcaa aaaagcggtt agctccttcg gtcctccgat cgttgtcaga agtaagttgg 840
ccgcagtgtt atcactcatg gttatggcag cactgcataa ttctcttact gtcatgccat 900
ccgtaagatg cttttctgtg actggtgagt actcaaccaa gtcattctga gaatagtgta 960
tgcggcgacc gagttgctct tgcccggcgt caatacggga taataccgcg ccacatagca 1020
gaactttaaa agtgctcatc attggaaaac gttcttcggg gcgaaaactc tcaaggatct 1080
taccgctgtt gagatccagt tcgatgtaac ccactcgtgc acccaactga tcttcagcat 1140
cttttacttt caccagcgtt tctgggtgag caaaaacagg aaggcaaaat gccgcaaaaa 1200
agggaataag ggcgacacgg aaatgttgaa tactcatact cttccttttt caatattatt 1260
gaagcattta tcagggttat tgtctcatga gcggatacat atttgaatgt atttagaaaa 1320
ataaacaaat aggggttccg cgcacatttc cccgaaaagt gccacctgac gtctaagaaa 1380
ccattattat catgacatta acctataaaa ataggcgtat cacgaggccc tttcgtctcg 1440
cgcgtttcgg tgatgacggt gaaaacctct gacacatgca gctcccggag acggtcacag 1500
cttgtctgta agcggatgcc gggagcagac aagcccgtca gggcgcgtca gcgggtgttg 1560
gcgggtgtcg gggctggctt aactatgcgg catcagagca gattgtactg agagtgcacc 1620
atatgcggtg tgaaataccg cacagatgcg taaggagaaa ataccgcatc aggcgccatt 1680
cgccattcag gctgcgcaac tgttgggaag ggcgatcggt gcgggcctct tcgctattac 1740
gccagctggc gaaaggggga tgtgctgcaa ggcgattaag ttgggtaacg ccagggtttt 1800
cccagtcacg acgttgtaaa acgacggcca gtgccaagct tgcatgcctg caggtcaatt 1860
ccctggcatt atgcccagta catgacctta tgggactttc ctacttggca gtacatctac 1920
gtattagtca tcgctattac catggtgatg cggttttggc agtacatcaa tgggcgtgga 1980
tagcggtttg actcacgggg atttccaagt ctccacccca ttgacgtcaa tgggagtttg 2040
ttttggcacc aaaatcaacg ggactttcca aaatgtcgta acaactccgc cccattgacg 2100

caaatgggcg gtaggcgtgt acggtgggag gtctatataa gcagagctcg tttagtgaac 2160
cgtcagatcg cctggagacg ccatccacgc tgttttgacc tccatagaag acaccgggac 2220
cgatccagcc tggggatcta gcctccgcgg ccgggaacgg tgcattggaa cgcggattcc 2280
ccgtgccaag agtgacgtaa gtaccgccta tagagtctat aggcccaccc ccttggcttc 2340
ttatgcatgc tatactgttt ttggcttggg gtctatacac ccccgcttcc tcatgttata 2400
ggtgatggta tagcttagcc tataggtgtg ggttattgac cattattgac cactccccta 2460
ttggtgacga tctttccatt actaatccat aacatggctc tttgccacaa ctctctttat 2520
tggctatatg ccaatacact gtccttcaga gactgacacg gactctgtat ttttacagga 2580
tggggtctca tttattattt acaaattcac atatacaaca ccaccgtccc cagtgcccgc 2640
agtttttatt aaacataacg tgggatctcc acgcgaatct cgggtacgtg ttccggacat 2700
gggctcttct ccggtagcgg cggagctcct acatccgagc cctgctccca tgcctccagc 2760
gactcatggt cgctcggcag ctccttgctc ctaacagtgg aggccagact taggcacagc 2820
acgatgccca ccaccaccag tgtgccgcac aaggccgtgg cggtagggta tgtgtctgaa 2880
aatgagctcg gggagcgggc ttgcaccgct gacgcatttg gaagacttaa ggcagcggca 2940
gaagaagatg caggcagctg agttgttgtg ttctgataag agtcagaggt aactcccgtt 3000
gcggtgctgt taacggtgga gggcagtgta gtctgagcag tactcgttgc tgccgcgcgc 3060
gccaccagac ataatagctg acagactaac agactgttcc tttccatggg tcttttctgc 3120
agtcaccgtc cttgacacga tcggatcccg ggtaccgagc tcggatccag tacccttcac 3180
cgacagagct cttctaaatg tatgttttat taacaattct aatctcggtt ctggtgtgtg 3240
aagcggttat tagagtgtct ctaagctcca caagagaaga gacctgcttt ggtgactcca 3300
ctaacccaga gatgattgaa ggagcttggg attcactcag agaggaggag atgccggagg 3360
agctctcctg ttctatatca ggcataagag aggttaagac ctcaagccag gagttataca 3420
gggcattaaa agccatcatt gctgctgatg gcttgaacaa catcacctgc catggtaagg 3480
atcctgagga caagatttcc ctcataaagg gtcctcctca caaaaagcgg gtggggatag 3540
ttcggtgtga gagacgaaga gatgctaagc aaatagggag agaaaccatg gcagggattg 3600
caatgacagt ccttccagcc ttagcagttt ttgctttggc acctgttgtt tttgctgaag 3660
acccccatct cagaaacaga ccagggaagg ggcacaacta cattgacggg atgactcagg 3720
aggatgccac atgcaaacct gtgacatatg ctggggcatg tagcagtttt gatgtcttgc 3780
ttgaaaaggg aaaatttccc cttttccagt cgtatgctca tcatagaact ctactagagg 3840
cagttcacga caccatcatt gcaaaggctg atccacctag ctgtgacctt ctgagtgctc 3900
atgggaaccc ctgcatgaaa gagaaactcg tgatgaagac acactgtcca aatgactacc 3960
agtcagctca ttacctcaac aatgacggga aaatggcttc agtcaagtgc cctcctaagt 4020
atgagctcac tgaggactgc aacttttgta ggcagatgac aggtgctagc ctgaagaagg 4080
ggtcttatcc tctccaagac ttgttttgtc agtcaagtga ggatgatgga tcaaaattaa 4140

aaacaaaaat gaaaggggtc tgcgaagtgg gggttcaagc actcaaaaag tgtgatggcc 4200
aactcagcac tgcacatgag gttgtgccct ttgcagtgtt taagaactca aagaaggttt 4260
atcttgataa gcttgacctt aagactgagg agaatctgct accagactca tttgtctgtt 4320
tcgagcataa gggacagtac aaaggaacaa tggactctgg tcagactaag agggagctca 4380
aaagctttga tatctctcag tgccccaaga ttggaggaca tggtagtaag aagtgcactg 4440
gggacgcagc attttgctct gcttatgagt gcactgctca gtacgccaat gcctattgtt 4500
cacatgctaa tgggtcaggg attgtgcaga tacaagtatc aggggtctgg aagaagcctt 4560
tatgtgtagg gtatgagaga gtggttgtga agagagaact ctctgccaag cccatccaga 4620
gagttgagcc ttgcacaact tgtataacca aatgtgagcc tcatggattg gttgtccgat 4680
caacagggtt caagatatca tcagcagttg cttgtgctag cggagtttgc gtcacaggat 4740
cacagagtcc ttccaccgag attacactca agtatccagg gatatcccag tcttctgggg 4800
gggacatagg ggttcacatg gcacacgatg atcagtcagt tagctccaaa atagtagctc 4860
actgccctcc ccaggacccg tgcttagtgc atgactgcat agtgtgtgct catggcctga 4920
taaattacca gtgtcacact gctctcagtg cctttgttgt tgtgtttgta ttcagttcta 4980
ttgcaataat ttgtttagct attctctata gggtgcttaa gtgcctgaag attgccccaa 5040
ggaaagttct gaatccacta atgtggatca cagccttcat cagatggata tataagaaga 5100
tggttgccag agtggcagac aacattaatc aagtgaacag ggaaatagga tggatggaag 5160
gaggtcagtt ggttctaggg aaccctgccc ctattcctcg tcatgcccca atcccacgtt 5220
atagcacata cctgatgtta ttattgattg tctcatatgc atcagcatgt tcagaactga 5280
ttcaggcaag ctccagaatc accacttgct ctacagaggg tgttaacacc aagtgtagac 5340
tgtctggcac agcattgatc agagcagggt cagttggggc agaggcttgt ttgatgttga 5400
agggggtcaa ggaagatcaa accaagttct taaagataaa aactgtctca agtgagctat 5460
catgcaggga gggccagagt tattggactg ggtcctttag ccctaaatgt ttgagctcaa 5520
ggagatgcca ccttgtcggg gaatgccatg tgaataggtg tctgtcttgg agggacaatg 5580
aaacttcagc agagttttca tttgttgggg aaagcacgac catgcgagag aataagtgtt 5640
ttgagcaatg tggaggatgg gggtgtgggt gtttcaatgt gaacccatct tgcttatttg 5700
tgcacacgta tctgcagtca gttagaaaag aggcccttag agtttttaac tgtatcgact 5760
gggtgcataa actcactcta gagatcacag actttgatgg ctctgtttca acaatagact 5820
tgggagcatc atctagccgt ttcacaaact ggggttcagt tagcctctca ctggacgcag 5880
agggcatttc aggctcaaat agcttttctt tcattgagag cccaggcaaa gggtatgcaa 5940
ttgttgatga gccattctca gaaattcctc ggcaagggtt cttgggggag atcaggtgca 6000
attcagagtc ctcagtcctg agtgctcatg aatcatgcct tagggcacca aaccttatct 6060
catacaagcc catgatagat caattggagt gcacaacaaa tctgattgat ccctttgttg 6120
tctttgagag gggttctctg ccacagacaa ggaatgacaa aacctttgca gcttcaaaag 6180

gaaatagagg tgttcaagct ttctctaagg gctctgtaca agctgatcta actctgatgt 6240
ttgacaattt tgaggtggac tttgtgggag cagccgtatc ttgtgatgcc gccttcttaa 6300
atttgacagg ttgctattct tgcaatgcag gggccagggt ctgcctgtct atcacatcca 6360
caggaactgg atccctctct gcccacaata aggatgggtc tctgcatata gtccttccat 6420
cagagaatgg aacaaaagac cagtgtcaga tactacactt cactgtgcct gaagtagagg 6480
aggagtttat gtactcttgt gatggagatg agcggcctct gttggtgaag gggaccctga 6540
tagccattga tccatttgat gataggcggg aagcaggggg ggaatcaaca gttgtgaatc 6600
caaaatctgg atcttggaat ttctttgact ggttttctgg actcatgagt tggtttggag 6660
ggcctcttaa aactatactc ctcatttgcc tgtatgttgc attatcaatt gggctctttt 6720
tcctccttat atatcttgga agaacaggcc tctctaaaat gtggcttgct gccactaaga 6780
aggcctcata gatcaggaag agctctgtca agggtcaaga caattctgca gatatccagc 6840
acagtggcgg ccgctcgagg aattctagat cccacgtcac tattgtatac tctatattat 6900
actctatgtt atactctgta atcctactca ataaacgtgt cacgcctgtg aaaccgtact 6960
aagtctcccg tgtcttctta tcaccatcag gtgacatcct cgcccaggct gtcaatcatg 7020
ccggtatcga ttccagtagc accggcccca cgctgacaac ccactcttgc agcgttagca 7080
gcgcccctct taacaagccg acccccacca gcgtcgcggt tactaacact cctctccccg 7140
acctgcagcc caagcttggc gtaatcatgg tcatagctgt ttcctgtgtg aaattgttat 7200
ccgctcacaa ttccacacaa catacgagcc ggaagcataa agtgtaaagc ctggggtgcc 7260
taatgagtga gctaactcac attaattgcg ttgcgctcac tgcccgcttt ccagtcggga 7320
aacctgtcgt gccagctgca ttaatgaatc ggccaacgcg cggggagagg cggtttgcgt 7380
attgggcgct cttccgcttc ctcgctcact gactcgctgc gctcggtcgt tcggctgcgg 7440
cgagcggtat cagctcactc aaaggcggta atacggttat ccacagaatc aggggataac 7500
gcaggaaaga acatgtgagc aaaaggccag caaaaggcca ggaaccgtaa aaaggccgcg 7560
ttgctggcgt ttttccatag gctccgcccc cctgacgagc atcacaaaaa tcgacgctca 7620
agtcagaggt ggcgaaaccc gacaggacta taaagatacc aggcgtttcc ccctggaagc 7680
tccctcgtgc gctctcctgt tccgaccctg ccgcttaccg gatacctgtc cgcctttctc 7740
ccttcgggaa gcgtggcgct ttctcatagc tcacgctgta ggtatctcag ttcggtgtag 7800
gtcgttcgct ccaagctggg ctgtgtgcac gaaccccccg ttcagcccga ccgctgcgcc 7860
ttatccggta actatcgtct tgagtccaac ccggtaagac acgacttatc gccactggca 7920
gcagccactg gtaacaggat tagcagagcg aggt 7954

<210> 27
<211> 7954
<212> DNA
<213> artificial sequence

<220>
<223> vector

<400> 27
atgtaggcgg tgctacagag ttcttgaagt ggtggcctaa ctacggctac actagaagaa 60
cagtatttgg tatctgcgct ctgctgaagc cagttacctt cggaaaaaga gttggtagct 120
cttgatccgg caaacaaacc accgctggta gcggtggttt ttttgtttgc aagcagcaga 180
ttacgcgcag aaaaaaagga tctcaagaag atcctttgat cttttctacg gggtctgacg 240
ctcagtggaa cgaaaactca cgttaaggga ttttggtcat gagattatca aaaaggatct 300
tcacctagat ccttttaaat taaaaatgaa gttttaaatc aatctaaagt atatatgagt 360
aaacttggtc tgacagttac caatgcttaa tcagtgaggc acctatctca gcgatctgtc 420
tatttcgttc atccatagtt gcctgactcc ccgtcgtgta gataactacg atacgggagg 480
gcttaccatc tggccccagt gctgcaatga taccgcgaga cccacgctca ccggctccag 540
atttatcagc aataaaccag ccagccggaa gggccgagcg cagaagtggt cctgcaactt 600
tatccgcctc catccagtct attaattgtt gccgggaagc tagagtaagt agttcgccag 660
ttaatagttt gcgcaacgtt gttgccattg ctacaggcat cgtggtgtca cgctcgtcgt 720
ttggtatggc ttcattcagc tccggttccc aacgatcaag gcgagttaca tgatccccca 780
tgttgtgcaa aaaagcggtt agctccttcg gtcctccgat cgttgtcaga agtaagttgg 840
ccgcagtgtt atcactcatg gttatggcag cactgcataa ttctcttact gtcatgccat 900
ccgtaagatg cttttctgtg actggtgagt actcaaccaa gtcattctga gaatagtgta 960
tgcggcgacc gagttgctct tgcccggcgt caatacggga taataccgcg ccacatagca 1020
gaactttaaa agtgctcatc attggaaaac gttcttcggg gcgaaaactc tcaaggatct 1080
taccgctgtt gagatccagt tcgatgtaac ccactcgtgc acccaactga tcttcagcat 1140
cttttacttt caccagcgtt tctgggtgag caaaaacagg aaggcaaaat gccgcaaaaa 1200
agggaataag ggcgacacgg aaatgttgaa tactcatact cttccttttt caatattatt 1260
gaagcattta tcagggttat tgtctcatga gcggatacat atttgaatgt atttagaaaa 1320
ataaacaaat aggggttccg cgcacatttc cccgaaaagt gccacctgac gtctaagaaa 1380
ccattattat catgacatta acctataaaa ataggcgtat cacgaggccc tttcgtctcg 1440
cgcgtttcgg tgatgacggt gaaaacctct gacacatgca gctcccggag acggtcacag 1500
cttgtctgta agcggatgcc gggagcagac aagcccgtca gggcgcgtca gcgggtgttg 1560
gcgggtgtcg gggctggctt aactatgcgg catcagagca gattgtactg agagtgcacc 1620
atatgcggtg tgaaataccg cacagatgcg taaggagaaa ataccgcatc aggcgccatt 1680
cgccattcag gctgcgcaac tgttgggaag ggcgatcggt gcgggcctct tcgctattac 1740
gccagctggc gaaaggggga tgtgctgcaa ggcgattaag ttgggtaacg ccagggtttt 1800
cccagtcacg acgttgtaaa acgacggcca gtgccaagct tgcatgcctg caggtcaatt 1860
ccctggcatt atgcccagta catgacctta tgggactttc ctacttggca gtacatctac 1920
gtattagtca tcgctattac catggtgatg cggttttggc agtacatcaa tgggcgtgga 1980

tagcggtttg actcacgggg atttccaagt ctccacccca ttgacgtcaa tgggagtttg 2040
ttttggcacc aaaatcaacg ggactttcca aaatgtcgta acaactccgc cccattgacg 2100
caaatgggcg gtaggcgtgt acggtgggag gtctatataa gcagagctcg tttagtgaac 2160
cgtcagatcg cctggagacg ccatccacgc tgttttgacc tccatagaag acaccgggac 2220
cgatccagcc tggggatcta gcctccgcgg ccgggaacgg tgcattggaa cgcggattcc 2280
ccgtgccaag agtgacgtaa gtaccgccta tagagtctat aggcccaccc ccttggcttc 2340
ttatgcatgc tatactgttt ttggcttggg gtctatacac ccccgcttcc tcatgttata 2400
ggtgatggta tagcttagcc tataggtgtg ggttattgac cattattgac cactccccta 2460
ttggtgacga tctttccatt actaatccat aacatggctc tttgccacaa ctctctttat 2520
tggctatatg ccaatacact gtccttcaga gactgacacg gactctgtat ttttacagga 2580
tggggtctca tttattattt acaaattcac atatacaaca ccaccgtccc cagtgcccgc 2640
agtttttatt aaacataacg tgggatctcc acgcgaatct cgggtacgtg ttccggacat 2700
gggctcttct ccggtagcgg cggagcttct acatccgagc cctgctccca tgcctccagc 2760
gactcatggt cgctcggcag ctccttgctc ctaacagtgg aggccagact taggcacagc 2820
acgatgccca ccaccaccag tgtgccgcac aaggccgtgg cggtagggta tgtgtctgaa 2880
aatgagctcg gggagcgggc ttgcaccgct gacgcatttg gaagacttaa ggcagcggca 2940
gaagaagatg caggcagctg agttgttgtg ttctgataag agtcagaggt aactcccgtt 3000
gcggtgctgt taacggtgga gggcagtgta gtctgagcag tactcgttgc tgccgcgcgc 3060
gccaccagac ataatagctg acagactaac agactgttcc tttccatggg tcttttctgc 3120
agtcaccgtc cttgacacga tcggatcccg ggtaccgagc tcggatccag tacccttcac 3180
cgacagagct cttctaaatg tatgttttat taacaattct aatctcggtt ctggtgtgtg 3240
aagcggttat tagagtgtct ctaagctcca caagagaaga gacctgcttt ggtgactcca 3300
ctaacccaga gatgattgaa ggagcttggg attcactcag agaggaggag atgccggagg 3360
agctctcctg ttctatatca ggcataagag aggttaagac ctcaagccag gagttataca 3420
gggcattaaa agccatcatt gctgctgatg gcttgaacaa catcacctgc catggtaagg 3480
atcctgagga caagatttcc ctcataaagg gtcctcctca caaaaagcgg gtggggatag 3540
ttcggtgtga gagacgaaga gatgctaagc aaatagggag agaaaccatg gcagggattg 3600
caatgacagt ccttccagcc ttagcagttt ttgctttggc acctgttgtt tttgctgaag 3660
acccccatct cagaaacaga ccagggaagg ggcacaacta cattgacggg atgactcagg 3720
aggatgccac atgcaaacct gtgacatatg ctggggcatg tagcagtttt gatgtcttgc 3780
ttgaaaaggg aaaatttccc cttttccagt cgtatgctca tcatagaact ctactagagg 3840
cagttcacga caccatcatt gcaaaggctg atccacctag ctgtgacctt ctgagtgctc 3900
atgggaaccc ctgcatgaaa gagaaactcg tgatgaagac acactgtcca aatgactacc 3960
agtcagctca ttacctcaac aatgacggga aaatggcttc agtcaagtgc cctcctaagt 4020

atgagctcac tgaggactgc aacttttgta ggcagatgac aggtgctagc ctgaagaagg 4080
ggtcttatcc tctccaagac ttgttttgtc agtcaagtga ggatgatgga tcaaaattaa 4140
aaacaaaaat gaaaggggtc tgcgaagtgg gggttcaagc actcaaaaag tgtgatggcc 4200
aactcagcac tgcacatgag gttgtgccct ttgcagtgtt taagaactca aagaaggttt 4260
atcttgataa gcttgacctt aagactgagg agaatctgct accagactca tttgtctgtt 4320
tcgagcataa gggacagtac aaaggaacaa tggactctgg tcagactaag agggagctca 4380
aaagctttga tatctctcag tgccccaaga ttggaggaca tggtagtaag aagtgcactg 4440
gggacgcagc attttgctct gcttatgagt gcactgctca gtacgccaat gcctattgtt 4500
cacatgctaa tgggtcaggg attgtgcaga tacaagtatc aggggtctgg aagaagcctt 4560
tatgtgtagg gtatgagaga gtggttgtga agagagaact ctctgccaag cccatccaga 4620
gagttgagcc ttgcacaact tgtataacca aatgtgagcc tcatggattg gttgtccgat 4680
caacagggtt caagatatca tcagcagttg cttgtgctag cggagtttgc gtcacaggat 4740
cacagagtcc ttccaccgag attacactca agtatccagg gatatcccag tcttctgggg 4800
gggacatagg ggttcacatg gcacacgatg atcagtcagt tagctccaaa atagtagctc 4860
actgccctcc ccaggacccg tgcttagtgc atgactgcat agtgtgtgct catggcctga 4920
taaattacca gtgtcacact gctctcagtg cctttgttgt tgtgtttgta ttcagttcta 4980
ttgcaataat ttgtttagct attctctata gggtgcttaa gtgcctgaag attgccccaa 5040
ggaaagttct gaatccacta atgtggatca cagccttcat cagatggata tataagaaga 5100
tggttgccag agtggcagac aacattaatc aagtgaacag ggaaatagga tggatggaag 5160
gaggtcagtt ggttctaggg aaccctgccc ctattcctcg tcatgcccca atcccacgtt 5220
atagcacata cctgatgtta ttattgattg tctcatatgc atcagcatgt tcagaactga 5280
ttcaggcaag ctccagaatc accacttgct ctacagaggg tgttaacacc aagtgtagac 5340
tgtctggcac agcattgatc agagcagggt cagttggggc agaggcttgt ttgatgttga 5400
agggggtcaa ggaagatcaa accaagttct taaagataaa aactgtctca agtgagctat 5460
catgcaggga gggccagagt tattggactg ggtcctttag ccctaaatgt ttgagctcaa 5520
ggagatgcca ccttgtcggg gaatgccatg tgaataggtg tctgtcttgg agggacaatg 5580
aaacttcagc agagttttca tttgttgggg aaagcacgac catgcgagag aataagtgtt 5640
ttgagcaatg tggaggatgg gggtgtgggt gtttcaatgt gaacccatct tgcttatttg 5700
tgcacacgta tctgcagtca gttagaaaag aggcccttag agtttttaac tgtatcgact 5760
gggtgcataa actcactcta gagatcacag actttgatgg ctctgtttca acaatagact 5820
tgggagcatc atctagccgt ttcacaaact ggggttcagt tagcctctca ctggacgcag 5880
agggcatttc aggctcaaat agcttttctt tcattgagag cccaggcaaa gggtatgcaa 5940
ttgttgatga gccattctca gaaattcctc ggcaagggtt cttgggggag atcaggtgca 6000
attcagagtc ctcagtcctg agtgctcatg aatcatgcct tagggcacca aaccttatct 6060

catacaagcc catgatagat caattggagt gcacaacaaa tctgattgat ccctttgttg 6120
tctttgagag gggttctctg ccacagacaa ggaatgacaa aacctttgca gcttcaaaag 6180
gaaatagagg tgttcaagct ttctctaagg gctctgtaca agctgatcta actctgatgt 6240
ttgacaattt tgaggtggac tttgtgggag cagccgtatc ttgtgatgcc gccttcttaa 6300
atttgacagg ttgctattct tgcaatgcag gggccagggt ctgcctgtct atcacatcca 6360
caggaactgg atccctctct gcccacaata aggatgggtc tctgcatata gtccttccat 6420
cagagaatgg aacaaaagac cagtgtcaga tactacactt cactgtgcct gaagtagagg 6480
aggagtttat gtactcttgt gatggagatg agcggcctct gttggtgaag gggaccctga 6540
tagccattga tccatttgat gataggcggg aagcaggggg ggaatcaaca gttgtgaatc 6600
caaaatctgg atcttggaat ttctttgact ggttttctgg actcatgagt tggtttggag 6660
ggcctcttaa aactatactc ctcatttgcc tgtatgttgc attatcaatt gggctctttt 6720
tcctccttat atatcttgga agaacaggcc tctctaaaat gtggcttgct gccactaaga 6780
aggcctcata gatcaggaag agctctgtca agggtcaaga caattctgca gatatccagc 6840
acagtggcgg ccgctcgagg aattctagat cccacgtcac tattgtatac tctatattat 6900
actctatgtt atactctgta atcctactca ataaacgtgt cacgcctgtg aaaccgtact 6960
aagtctcccg tgtcttctta tcaccatcag gtgacatcct cgcccaggct gtcaatcatg 7020
ccggtatcga ttccagtagc accggcccca cgctgacaac ccactcttgc agcgttagca 7080
gcgcccctct taacaagccg acccccacca gcgtcgcggt tactaacact cctctccccg 7140
acctgcagcc caagcttggc gtaatcatgg tcatagctgt ttcctgtgtg aaattgttat 7200
ccgctcacaa ttccacacaa catacgagcc ggaagcataa agtgtaaagc ctggggtgcc 7260
taatgagtga gctaactcac attaattgcg ttgcgctcac tgcccgcttt ccagtcggga 7320
aacctgtcgt gccagctgca ttaatgaatc ggccaacgcg cggggagagg cggtttgcgt 7380
attgggcgct cttccgcttc ctcgctcact gactcgctgc gctcggtcgt tcggctgcgg 7440
cgagcggtat cagctcactc aaaggcggta atacggttat ccacagaatc aggggataac 7500
gcaggaaaga acatgtgagc aaaaggccag caaaaggcca ggaaccgtaa aaaggccgcg 7560
ttgctggcgt ttttccatag gctccgcccc cctgacgagc atcacaaaaa tcgacgctca 7620
agtcagaggt ggcgaaaccc gacaggacta taaagatacc aggcgtttcc ccctggaagc 7680
tccctcgtgc gctctcctgt tccgaccctg ccgcttaccg gatacctgtc cgcctttctc 7740
ccttcgggaa gcgtggcgct ttctcatagc tcacgctgta ggtatctcag ttcggtgtag 7800
gtcgttcgct ccaagctggg ctgtgtgcac gaaccccccg ttcagcccga ccgctgcgcc 7860
ttatccggta actatcgtct tgagtccaac ccggtaagac acgacttatc gccactggca 7920
gcagccactg gtaacaggat tagcagagcg aggt 7954

<210> 28
<211> 7954
<212> DNA

<213> artificial sequence

<220>
<223> vector

<400> 28
atgtaggcgg tgctacagag ttcttgaagt ggtggcctaa ctacggctac actagaagaa 60

cagtatttgg tatctgcgct ctgctgaagc cagttacctt cggaaaaaga gttggtagct 120

cttgatccgg caaacaaacc accgctggta gcggtggttt ttttgtttgc aagcagcaga 180

ttacgcgcag aaaaaaagga tctcaagaag atcctttgat cttttctacg gggtctgacg 240

ctcagtggaa cgaaaactca cgttaaggga ttttggtcat gagattatca aaaaggatct 300

tcacctagat ccttttaaat taaaaatgaa gttttaaatc aatctaaagt atatatgagt 360

aaacttggtc tgacagttac caatgcttaa tcagtgaggc acctatctca gcgatctgtc 420

tatttcgttc atccatagtt gcctgactcc ccgtcgtgta gataactacg atacgggagg 480

gcttaccatc tggccccagt gctgcaatga taccgcgaga cccacgctca ccggctccag 540

atttatcagc aataaaccag ccagccggaa gggccgagcg cagaagtggt cctgcaactt 600

tatccgcctc catccagtct attaattgtt gccgggaagc tagagtaagt agttcgccag 660

ttaatagttt gcgcaacgtt gttgccattg ctacaggcat cgtggtgtca cgctcgtcgt 720

ttggtatggc ttcattcagc tccggttccc aacgatcaag gcgagttaca tgatccccca 780

tgttgtgcaa aaaagcggtt agctccttcg gtcctccgat cgttgtcaga agtaagttgg 840

ccgcagtgtt atcactcatg gttatggcag cactgcataa ttctcttact gtcatgccat 900

ccgtaagatg cttttctgtg actggtgagt actcaaccaa gtcattctga gaatagtgta 960

tgcggcgacc gagttgctct tgcccggcgt caatacggga taataccgcg ccacatagca 1020

gaactttaaa agtgctcatc attggaaaac gttcttcggg gcgaaaactc tcaaggatct 1080

taccgctgtt gagatccagt tcgatgtaac ccactcgtgc acccaactga tcttcagcat 1140

cttttacttt caccagcgtt tctgggtgag caaaaacagg aaggcaaaat gccgcaaaaa 1200

agggaataag ggcgacacgg aaatgttgaa tactcatact cttccttttt caatattatt 1260

gaagcattta tcagggttat tgtctcatga gcggatacat atttgaatgt atttagaaaa 1320

ataaacaaat aggggttccg cgcacatttc cccgaaaagt gccacctgac gtctaagaaa 1380

ccattattat catgacatta acctataaaa ataggcgtat cacgaggccc tttcgtctcg 1440

cgcgtttcgg tgatgacggt gaaaacctct gacacatgca gctcccggag acggtcacag 1500

cttgtctgta agcggatgcc gggagcagac aagcccgtca gggcgcgtca gcgggtgttg 1560

gcgggtgtcg gggctggctt aactatgcgg catcagagca gattgtactg agagtgcacc 1620

atatgcggtg tgaaataccg cacagatgcg taaggagaaa ataccgcatc aggcgccatt 1680

cgccattcag gctgcgcaac tgttgggaag ggcgatcggt gcgggcctct tcgctattac 1740

gccagctggc gaaaggggga tgtgctgcaa ggcgattaag ttgggtaacg ccagggtttt 1800

cccagtcacg acgttgtaaa acgacggcca gtgccaagct tgcatgcctg caggtcaatt 1860

ccctggcatt atgcccagta catgacctta tgggactttc ctacttggca gtacatctac 1920
gtattagtca tcgctattac catggtgatg cggttttggc agtacatcaa tgggcgtgga 1980
tagcggtttg actcacgggg atttccaagt ctccacccca ttgacgtcaa tgggagtttg 2040
ttttggcacc aaaatcaacg ggactttcca aaatgtcgta acaactccgc cccattgacg 2100
caaatgggcg gtaggcgtgt acggtgggag gtctatataa gcagagctcg tttagtgaac 2160
cgtcagatcg cctggagacg ccatccacgc tgttttgacc tccatagaag acaccgggac 2220
cgatccagcc tggggatcta gcctccgcgg ccgggaacgg tgcattggaa cgcggattcc 2280
ccgtgccaag agtgacgtaa gtaccgccta tagagtctat aggcccaccc ccttggcttc 2340
ttatgcatgc tatactgttt ttggcttggg gtctatacac ccccgcttcc tcatgttata 2400
ggtgatggta tagcttagcc tataggtgtg ggttattgac cattattgac cactccccta 2460
ttggtgacga tctttccatt actaatccat aacatggctc tttgccacaa ctctctttat 2520
tggctatatg ccaatacact gtccttcaga gactgacacg gactctgtat ttttacagga 2580
tggggtctca tttattattt acaaattcac atatacaaca ccaccgtccc cagtgcccgc 2640
agtttttatt aaacataacg tgggatctcc acgcgaatct cgggtacgtg ttccggacat 2700
gggctcttct ccggtagcgg cggagctcct acatccgagc cctgctccca tgcctccagc 2760
gactcatggt cgctcggcag ctccttgctc ctaacagtgg aggccagact taggcacagc 2820
acgatgccca ccaccaccag tgtgccgcac aaggccgtgg cggtagggta tgtgtctgaa 2880
aatgagctcg gggagcgggc ttgcaccgct gacgcatttg gaagacttaa ggcagcggca 2940
gaagaagatg caggcagctg agttgttgtg ttctgataag agtcagaggt aactcccgtt 3000
gcggtgctgt taacggtgga gggcagtgta gtctgagcag tactcgttgc tgccgcgcgc 3060
gccaccagac ataatagctg acagactaac agactgttcc tttccatggg tcttttctgc 3120
agtcaccgtc cttgacacga tcggatcccg ggtaccgagc tcggatccag tacccttcac 3180
cgacagagct cttctaaatg tatgttttat taacaattct aatctcggtt ctggtgtgtg 3240
aagcggttat tagagtgtct ctaagctcca caagagaaga gacctgcttt ggtgactcca 3300
ctaacccaga gatgattgaa ggagcttggg attcactcag agaggaggag atgccggagg 3360
agctctcctg ttctatatca ggcataagag aggttaagac ctcaagccag gagttataca 3420
gggcattaaa agccatcatt gctgctgatg gcttgaacaa catcacctgc catggtaagg 3480
atcctgagga caagatttcc ctcataaagg gtcctcctca caaaaagcgg gtggggatag 3540
ttcggtgtga gagacgaaga gatgctaagc aaatagggag agaaaccatg gcagggattg 3600
caatgacagt ccttccagcc ttagcagttt ttgctttggc acctgttgtt tttgctgaag 3660
acccccatct cagaaacaga ccagggaagg ggcacaacta cattgacggg atgactcagg 3720
aggatgccac atgcaaacct gtgacatatg ctggggcatg tagcagtttt gatgtcttgc 3780
ttgaaaaggg aaaatttccc cttttccagt cgtatgctca tcatagaact ctactagagg 3840
cagttcacga caccatcatt gcaaaggctg atccacctag ctgtgacctt ctgagtgctc 3900

atgggaaccc ctgcatgaaa gagaaactcg tgatgaagac acactgtcca aatgactacc 3960
agtcagctca ttacctcaac aatgacggga aaatggcttc agtcaagtgc cctcctaagt 4020
atgagctcac tgaggactgc aacttttgta ggcagatgac aggtgctagc ctgaagaagg 4080
ggtcttatcc tctccaagac ttgttttgtc agtcaagtga ggatgatgga tcaaaattaa 4140
aaacaaaaat gaaaggggtc tgcgaagtgg gggttcaagc actcaaaaag tgtgatggcc 4200
aactcagcac tgcacatgag gttgtgccct ttgcagtgtt taagaactca aagaaggttt 4260
atcttgataa gcttgacctt aagactgagg agaatctgct accagactca tttgtctgtt 4320
tcgagcataa gggacagtac aaaggaacaa tggactctgg tcagactaag agggagctca 4380
aaagctttga tatctctcag tgccccaaga ttggaggaca tggtagtaag aagtgcactg 4440
gggacgcagc attttgctct gcttatgagt gcactgctca gtacgccaat gcctattgtt 4500
cacatgctaa tgggtcaggg attgtgcaga tacaagtatc aggggtctgg aagaagcctt 4560
tatgtgtagg gtatgagaga gtggttgtga agagagaact ctctgccaag cccatccaga 4620
gagttgagcc ttgcacaact tgtataacca aatgtgagcc tcatggattg gttgtccgat 4680
caacagggtt caagatatca tcagcagttg cttgtgctag cggagtttgc gtcacaggat 4740
cacagagtcc ttccaccgag attacactca agtatccagg gatatcccag tcttctgggg 4800
gggacatagg ggttcacatg gcacacgatg atcagtcagt tagctccaaa atagtagctc 4860
actgccctcc ccaggacccg tgcttagtgc atgactgcat agtgtgtgct catggcctga 4920
taaattacca gtgtcacact gctctcagtg cctttgttgt tgtgtttgta ttcagttcta 4980
ttgcaataat ttgtttagct attctctata gggtgcttaa gtgcctgaag attgccccaa 5040
ggaaagttct gaatccacta atgtggatca cagccttcat cagatggata tataagaaga 5100
tggttgccag agtggcagac aacattaatc aagtgaacag ggaaatagga tggatggaag 5160
gaggtcagtt ggttctaggg aaccctgccc ctattcctcg tcatgcccca atcccacgtt 5220
atagcacata cctgatgtta ttattgattg tctcatatgc atcagcatgt tcagaactga 5280
ttcaggcaag ctccagaatc accacttgct ctacagaggg tgttaacacc aagtgtagac 5340
tgtctggcac agcattgatc agagcagggt cagttggggc agaggcttgt ttgatgttga 5400
agggggtcaa ggaagatcaa accaagttct taaagataaa aactgtctca agtgagctat 5460
catgcaggga gggccagagt tattggactg ggtcctttag ccctaaatgt ttgagctcaa 5520
ggagatgcca ccttgtcggg gaatgccatg tgaataggtg tctgtcttgg agggacaatg 5580
aaacttcagc agagttttca tttgttgggg aaagcacgac catgcgagag aataagtgtt 5640
ttgagcaatg tggaggatgg gggtgtgggt gtttcaatgt gaacccatct tgcttatttg 5700
tgcacacgta tctgcagtca gttagaaaag aggcccttag agtttttaac tgtatcgact 5760
gggtgcataa actcactcta gagatcacag actttgatgg ctctgtttca acaatagact 5820
tgggagcatc atctagccgt ttcacaaact ggggttcagt tagcctctca ctggacgcag 5880
agggcatttc aggctcaaat agcttttctt tcattgagag cccaggcaaa gggtatgcaa 5940

ttgttgatga gccattctca gaaattcctc ggcaagggtt cttgggggag atcaggtgca 6000
attcagagtc ctcagtcctg agtgctcatg aatcatgcct tagggcacca aaccttatct 6060
catacaagcc catgatagat caattggagt gcacaacaaa tctgattgat ccctttgttg 6120
tctttgagag gggttctctg ccacagacaa ggaatgacaa aacctttgca gcttcaaaag 6180
gaaatagagg tgttcaagct ttctctaagg gctctgtaca agctgatcta actctgatgt 6240
ttgacaattt tgaggtggac tttgtgggag cagccgtatc ttgtgatgcc gccttcttaa 6300
atttgacagg ttgctattct tgcaatgcag gggccagggt ctgcctgtct atcacatcca 6360
caggaactgg atccctctct gcccacaata aggatgggtc tctgcatata gtccttccat 6420
cagagaatgg aacaaaagac cagtgtcaga tactacactt cactgtgcct gaagtagagg 6480
aggagtttat gtactcttgt gatggagatg agcggcctct gttggtgaag gggaccctga 6540
tagccattga tccatttgat gataggcggg aagcaggggg ggaatcaaca gttgtgaatc 6600
caaaatctgg atcttggaat ttctttgact ggttttctgg actcatgagt tggtttggag 6660
ggcctcttaa aactatactc ctcatttgcc tgtatgttgc attatcaatt gggctctttt 6720
tcctccttat atatcttgga agaacaggcc tctctaaaat gtggcttgct gccactaaga 6780
aggcctcata gatcaggaag agctctgtca agggtcaaga caattctgca gatatccagc 6840
acagtggcgg ccgctcgagg aattctagat cccacgtcac tattgtatac tctatattat 6900
actctatgtt atactctgta atcctactca ataaacgtgt cacgcctgtg aaaccgtact 6960
aagtctcccg tgtcttctta tcaccatcag gtgacatcct cgcccaggct gtcaatcatg 7020
ccggtatcga ttccagtagc accggcccca cgctgacaac ccactcttgc agcgttagca 7080
gcgcccctct taacaagccg acccccacca gcgtcgcggt tactaacact cctctccccg 7140
acctgcagcc caagcttggc gtaatcatgg tcatagctgt ttcctgtgtg aaattgttat 7200
ccgctcacaa ttccacacaa catacgagcc ggaagcataa agtgtaaagc ctggggtgcc 7260
taatgagtga gctaactcac attaattgcg ttgcgctcac tgcccgcttt ccagtcggga 7320
aacctgtcgt gccagctgca ttaatgaatc ggccaacgcg cggggagagg cggtttgcgt 7380
attgggcgct cttccgcttc ctcgctcact gactcgctgc gctcggtcgt tcggctgcgg 7440
cgagcggtat cagctcactc aaaggcggta atacggttat ccacagaatc aggggataac 7500
gcaggaaaga acatgtgagc aaaaggccag caaaaggcca ggaaccgtaa aaaggccgcg 7560
ttgctggcgt ttttccatag gctccgcccc cctgacgagc atcacaaaaa tcgacgctca 7620
agtcagaggt ggcgaaaccc gacaggacta taaagatacc aggcgtttcc ccctggaagc 7680
tccctcgtgc gctctcctgt tccgaccctg ccgcttaccg gatacctgtc cgcctttctc 7740
ccttcgggaa gcgtggcgct ttctcatagc tcacgctgta ggtatctcag ttcggtgtag 7800
gtcgttcgct ccaagctggg ctgtgtgcac gaaccccccg ttcagcccga ccgctgcgcc 7860
ttatccggta actatcgtct tgagtccaac ccggtaagac acgacttatc gccactggca 7920
gcagccactg gtaacaggat tagcagagcg aggt 7954